(19) Europäisches Patentamt
European Patent Office
Office européen des brevets

(11) EP 3 587 456 B1

(12) **EUROPÄISCHE PATENTSCHRIFT**

(45) Veröffentlichungstag und Bekanntmachung des Hinweises auf die Patenterteilung:
**25.02.2026 Patentblatt 2026/09**

(21) Anmeldenummer: **18179037.9**

(22) Anmeldetag: **21.06.2018**

(51) Internationale Patentklassifikation (IPC):
***C08B 37/08*** *(2006.01)* ***A61K 31/722*** *(2006.01)*

(52) Gemeinsame Patentklassifikation (CPC):
**C08B 37/003; A61K 31/722**

(54) **VERFAHREN ZUR HERSTELLUNG EINES ANTIBAKTERIELLEN CHITOSANHALTIGEN POLYMERS FÜR MEDIZINISCHE ZWECKE, INSBESONDERE FÜR DIE WUNDBEHANDLUNG**

METHOD FOR THE PRODUCTION OF ANTIBACTERIAL CHITOSANE-CONTAINING POLYMER FOR MEDICAL PURPOSES, IN PARTICULAR FOR TREATING WOUNDS

PROCÉDÉ DE FABRICATION D’UN POLYMÈRE ANTIBACTÉRIEN CONTENANT DU CHITOSANE À USAGE MÉDICALE, NOTAMMENT POUR LE TRAITEMENT DES PLAIES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**01.01.2020 Patentblatt 2020/01**

(73) Patentinhaber: **Paul Hartmann AG**
**89522 Heidenheim (DE)**

(72) Erfinder:
- **KETTEL, Dr. Markus**
  **89520 Heidenheim (DE)**
- **TAMULAITYTE, Rasa Irena**
  **5311 Bonn (DE)**

(74) Vertreter: **Paul Hartmann AG**
**Patents & Licensing**
**Paul-Hartmann-Straße 12**
**89522 Heidenheim (DE)**

(56) Entgegenhaltungen:
- **VELAZQUEZ-MORALES P ET AL: "Polymer electrolytes derived from chitosan/polyether networks", ELECTROCHIMICA ACTA, ELSEVIER SCIENCE PUBLISHERS, BARKING, GB, vol. 43, no. 10-11, 30 April 1998 (1998-04-30), pages 1275 - 1279, XP027538749, ISSN: 0013-4686, [retrieved on 19980430]**
- **GANDINI A ET AL: "Recent Contributions to the Preparaion of Polymers Derived from Renewable Resources", JOURNAL OF POLYMERS AND THE ENVIRONMENT, SPRINGER NEW YORK LLC, US, vol. 10, no. 3, 1 July 2002 (2002-07-01), pages 105 - 114, XP002412949, ISSN: 1566-2543, DOI: 10.1023/A:1021172130748**
- **LEVI-POLYACHENKO NICOLE ET AL: "Chitosan wound dressing with hexagonal silver nanoparticles for hyperthermia and enhanced delivery of small molecules", COLLOIDS AND SURFACES. B, BIOINTERFACES, ELSEVIER, AMSTERDAM, NL, vol. 142, 18 February 2016 (2016-02-18), pages 315 - 324, XP029501259, ISSN: 0927-7765, DOI: 10.1016/ J.COLSURFB.2016.02.038**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 3 587 456 B1

**Beschreibung**

Die vorliegende Erfindung betrifft ein Verfahren zur Herstellung eines Polymers für medizinische Zwecke, insbesondere für die Wundbehandlung. Die Erfindung betrifft auch das mit dem Verfahren erhaltene Polymer als solches sowie dessen Einsatz in der Medizin und in Wundauflagen.

Wundverbände mit einer Hydrogelschicht sind im Stand der Technik bekannt. Derartige Wundverbände können sowohl Wundexsudat absorbieren als auch die Wunde befeuchten und so die Wundheilung beschleunigen.

Wundverbände mit einer antibakteriellen Komponente sind ebenfalls im Stand der Technik bekannt. Aufgrund ihrer antibakteriellen Eigenschaften werden sie insbesondere zur Behandlung von infizierten Wunden eingesetzt.

Velazquez-Morales et al. ("Polymer electrolytes derived from chitosan/polyether networks"; Electrochimica Acta, Bd. 43, 1998) beschreiben die Synthese von oxipropyliertem Chitosan und dessen Reaktion mit Oligoether-basierten Mono- und Diisocyanaten. Ziel ist die Verbesserung von quervernetzten Polymerelektrolyten im Hinblick auf deren filmbildende Eigenschaften, um besonders dünne Polymerfilme herstellen zu können.

Gandini et al. ("Recent contributions to the preparation of polymers derived from renewable resources"; Journal of Polymers and the Environment, Bd. 10, 2002) befassen sich mit der Verwendung von erneuerbaren Ressourcen als Startmaterialien für die Synthese von Polymeren. Dabei wird die Reaktion von oxipropyliertem Chitosan mit einem Isocyanat-terminierten Präpolymer offenbart.

Levi-Polyachenko et al. ("Chitosan wound dressing with hexagonal silver nanoparticles for hyperthermia and enhanced delivery of small molecules"; Colloids and Surfaces B: Biointerfaces, Bd. 142, 2016) offenbaren eine Wundauflage, bei der es sich um einen getrockneten Film bestehend aus Chitosan und Silbernanopartikeln handelt.

Es besteht das Bedürfnis, neue Materialien für Wundverbände auf Basis von Hydrogelen bereitzustellen.

Aufgabe der vorliegenden Erfindung war es, ein neues und verbessertes Material für medizinische Zwecke bereitzustellen. Insbesondere war es die Aufgabe der vorliegenden Erfindung, ein neues und verbessertes Material für die Wundbehandlung zur Verfügung zu stellen. Dabei wurden an das Material verschiedene Anforderungen gestellt. Diese Anforderungen sahen wie folgt aus:

a) Das Material soll eine gute antibakterielle Wirksamkeit aufweisen.
b) Das Material soll keine antibakteriellen Komponenten freisetzen.
c) Das Material soll für eine Vielzahl unterschiedlicher Wundtypen verwendbar sein und eine schonende Wundbehandlung ermöglichen.
d) Das Material soll einfach und schnell herstellbar sein.
e) Das Material soll keine für den menschlichen oder tierischen Körper toxische Substanzen enthalten.

Es hat sich gezeigt, dass ein Polymer, hergestellt mit dem nachfolgenden Verfahren, die zuvor genannten Aufgaben und Anforderungen erfüllen kann.

Die vorliegende Erfindung ist in den beiliegenden Ansprüchen definiert.

Das erfindungsgemäße Polymer zur Anwendung in der Wundbehandlung im Sinne einer medizinischen Indikation gemäß Anspruch 1 sowie als Bestandteil eines Wundverbands gemäß Anspruch 2 wird mit den nachfolgenden Schritten hergestellt:

i. Bereitstellen eines Isocyanat-terminierten Präpolymers enthaltend Polyalkylenoxideinheiten.
ii. Lösen eines Chitosans in einer wässrigen Flüssigkeit, um eine wässrige, chitosanhaltige Zubereitung zu erhalten.
iii. Mischen des Präpolymers, der wässrigen, chitosanhaltigen Zubereitung und optional einer oder mehrerer weiterer Komponenten, um eine Reaktionsmischung zu erhalten.
iv. Umsetzen des Präpolymers in der Reaktionsmischung, um das Polymer zu erhalten.

Dabei ist der Anteil an dem Präpolymer, dem Chitosan, der wässrigen Flüssigkeit und der einen oder mehreren weiteren Komponenten, jeweils bezogen auf das Gesamtgewicht der Reaktionsmischung, wie folgt: 5 bis 50 Gew.-% Präpolymer, mindestens 0,4 Gew.-% Chitosan, 40 bis 90 Gew.-% der wässrigen Flüssigkeit und 0 bis 30 Gew.-% der einen oder mehreren weiteren Komponenten.

Die Reihenfolge der Schritte i. und ii. kann auch vertauscht werden und ist nicht erfindungswesentlich.

Das Isocyanat-terminierte Präpolymer wird im vorliegenden Dokument auch verkürzt als Präpolymer bezeichnet. Wird also im vorliegenden Dokument auf das Präpolymer Bezug genommen, so ist insofern nicht anders angegeben das Isocyanat-terminierte Präpolymer gemeint.

Kennzeichnend für die vorliegende Erfindung ist, dass das Chitosan in das Polymer eingeschlossen ist. Dies wird insbesondere dadurch erreicht, indem das Präpolymer in der Reaktionsmischung mit dem Chitosan reagieren kann und somit in dem Polymer zumindest ein Teil des Chitosans oder vorzugsweise die Gesamtmenge des Chitosans kovalent gebunden vorliegt. Die hierbei ablaufenden Reaktionsmechanismen werden nachfolgend noch genauer beschrieben.

Grundsätzlich wird mit dem erfindungsgemäßen Polymer ein hydratisiertes Polyurethan-Hydrogel-System beziehungsweise ein hydratisiertes Polyurethan-Schaum-System bereitgestellt, in dessen quervernetzte Polymerstruktur Chitosan insbesondere durch kovalenten Einbau fest eingeschlossen ist. Dadurch werden die Vorzüge der Hydrogeltechnologie in der Wundbehandlung mit den antibakteriellen Eigenschaften des Chitosans auf besonders vorteilhafte Art und Weise miteinander kombiniert. So kann das erfindungsgemäße Polymer ein die Wundheilung unterstützendes feuchtes Wundklima erzeugen, indem es Wundexsudat absorbieren und/oder Feuchtigkeit an die Wunde abgeben kann. Es verklebt dabei nicht mit der Wunde. Bereits diese Eigenschaften ermöglichen eine effektive und schonende Behandlung von vielen unterschiedlichen Wundtypen. Zudem besitzt das erfindungsgemäße Polymer durch seinen Gehalt an Chitosan eine antibakterielle Wirksamkeit, was sein wundheilungsförderndes Potential weiter verbessert. Dabei wird es als besonders vorteilhaft angesehen, dass das Chitosan als antibakterielle Wirkkomponente fest in der Polymerstruktur eingeschlossen ist und folglich nicht freigesetzt wird. Das Polymer gibt das Chitosan also nicht an die Wunde ab, was dessen antibakterielle Wirksamkeit herabsetzen könnte. Weitere Vorteile des erfindungsgemäßen Polymers sind seine einfache und schnelle Herstellbarkeit sowie seine gute Verträglichkeit für menschliche und tierische Gewebe.

In einer besonders bevorzugten Ausführungsform der Erfindung ist das Präpolymer mindestens dreiarmig (insbesondere genau dreiarmig) verzweigt und die Polyalkylenoxideinheiten werden durch Polyethylenoxid- und/oder Polypropylenoxideinheiten gebildet. Im obigen Schritt i. wird dann also ein mindestens dreiarmig verzweigtes Isocyanat-terminiertes Präpolymer enthaltend Polyethylenoxid- und/oder Polypropylenoxideinheiten bereitgestellt. Dabei kann das Gewichtsverhältnis von Ethylenoxid- zu Propylenoxideinheiten 3 : 1 bis 7 : 1 betragen. Mit einer solchen Verbindung lässt sich das Polymer besonders gut herstellen.

Ein typisches Isocyanat-terminiertes Präpolymer gemäß der zuvor genannten besonders bevorzugten Ausführungsform ist beispielsweise ein dreiarmiges Copolymer aus Ethylenoxid- und Propylenoxideinheiten, welches endständig jeweils mit einem Molekül Isophorondiisocyanat umgesetzt wurde. Es weist einen Gehalt reaktiver Isocyanatendgruppen (NCO-Gruppen) von 2,5 % bis 4,0 %, bevorzugt 3,0 % bis 3,4 %, besonders bevorzugt 3,2 %, und ein molares Verhältnis von Ethylenoxideinheiten zu Propylenoxideinheiten von 3 : 1 bis 4 : 1 auf. Ein derartiges Isocyanat-terminiertes Präpolymer mit aliphatischen Isocyanat-Gruppen ist zum Beispiel als Aquapol® PI-13000-31 (Carpenter; Richmond, USA) kommerziell erhältlich.

Die nachfolgende Abbildung veranschaulicht die schematische Struktur eines dreiarmig verzweigten Isocyanat-terminierten Präpolymers enthaltend Polyethylenoxid- und Polypropylenoxideinheiten (wie bei Aquapol®). Ein Glycerol-Molekül bildet das Zentrum des Präpolymers. Die drei "Arme" des Präpolymers mit jeweils einer endständigen Isocyanat-Gruppe sind mit den Hydroxyl-Gruppen des Glycerol-Moleküles verknüpft. Das Glycerol-Molekül selber ist in der Abbildung nicht dargestellt. Es würde sich in der rechten Bildhälfte dort befinden, wo die als Wellenlinie schematisiert dargestellten drei "Arme" aufeinandertreffen. In der linken Bildhälfte ist die chemische Struktur eines "Arms" genauer dargestellt.

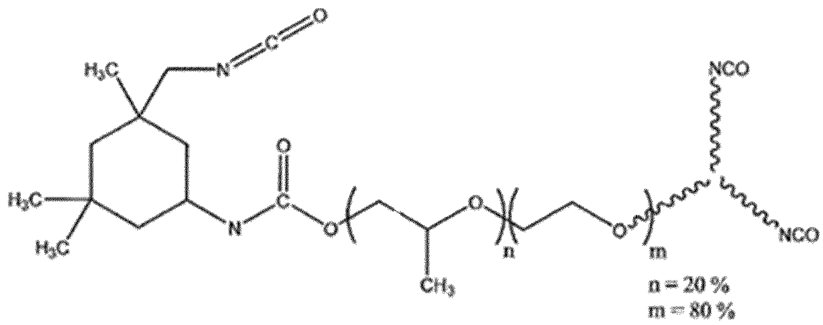

n = 20 %
m = 80 %

Chitosan ist in verschiedenen molaren Gewichten verfügbar und wird üblicherweise aus Chitin durch Deacetylierung gewonnen. Das Chitosan kann ein molares Gewicht von 50 kDa bis 375 kDa, bevorzugt von 50 kDa bis 310 kDa und besonders bevorzugt von 50 kDa bis 190 kDa aufweisen. Ein niedrigeres molares Gewicht hat den Vorteil, dass das Chitosan leichter gelöst werden kann. Zudem kann das Chitosan einen Deacetylierungsgrad von mindestens 50 %, bevorzugt von mindestens 60 %, mehr bevorzugt von mindestens 70 % und besonders bevorzugt von mindestens 75 % aufweisen. Insbesondere weist das Chitosan einen Deacetylierungsgrad von 75 % bis 85 % auf. Ein Chitosan mit einem hohen Deacetylierungsgrad besitzt viele hochreaktive Amin-Gruppen für ein schnelles Umsetzen des Präpolymers. Entsprechend wird vorliegend ein Chitosan mit einem molaren Gewicht von 50 kDa bis 190 kDa und einem Deacetylierungsgrad von mindestens 75 %, insbesondere 75 % bis 85 %, besonders bevorzugt. Ein derartiges Chitosan ist von der Firma Sigma-Aldrich als "low molecular weight chitosan" im Handel erhältlich.

In der Regel weist die wässrige Flüssigkeit einen sauren pH-Wert auf, da das Chitosan andernfalls nicht oder nur schlecht gelöst werden kann. Eine wässrige Flüssigkeit hat einen Wassergehalt von mindestens 50 Gew.-%. Normalerweise hat die vorliegend eingesetzte wässrige Flüssigkeit jedoch einen deutlich höheren Wassergehalt, zum Beispiel

mindestens 80 Gew.-%, mindestens 85 Gew.-%, mindestens 90 Gew.-% oder mindestens 95 Gew.-%. Ein pH-Wert ist sauer, wenn er kleiner als 7 ist. Der pH-Wert der wässrigen Flüssigkeit kann beispielsweise 0 bis 6, 0 bis 5, 0 bis 4, 0 bis 3, 0 bis 2, 0 bis 1, 1 bis 6, 2 bis 6, 3 bis 6, 4 bis 6 oder 5 bis 6 sein. Umso kleiner der pH-Wert der wässrigen Flüssigkeit ist, umso mehr Chitosan kann gelöst werden. Umso schwächer sauer die wässrige Flüssigkeit ist, umso besser können die Amin-Gruppen des gelösten Chitosans mit dem Präpolymer reagieren. Insbesondere handelt es sich bei der wässrigen Flüssigkeit um verdünnte Salzsäure.

Grundsätzlich handelt es sich bei der wässrigen Flüssigkeit um Wasser, dem eine Säure wie zum Beispiel Salzsäure zugesetzt ist. Die Menge an zugesetzter Säure hängt von der Menge des zu lösenden Chitosans ab. So steigt die Säurekonzentration in der Flüssigkeit üblicherweise bei Erhöhung der zu lösenden Chitosanmenge an. Neben Salzsäure können auch andere starke anorganische oder organische Säuren eingesetzt werden, um das Chitosan in Lösung zu bringen. Beispiele hierfür sind Ameisensäure, Citronensäure, Essigsäure, Milchsäure, Phosphorsäure sowie Propansäure. Jedoch werden vorliegend anorganische Säuren gegenüber organischen Säuren bevorzugt, weil sie fast keine Rückstände in dem Polymer hinterlassen. Die hergestellten Polymere weisen dann also eine höhere Reinheit auf. Zum Beispiel hinterlässt die Salzsäure nur inerte Chloridionen in dem Polymer.

Gegebenenfalls kann die wässrige Flüssigkeit neben der Säurekomponente auch noch andere Stoffe enthalten. Insbesondere ist vorliegend aber vorgesehen, dass die wässrige Flüssigkeit abgesehen von der Säurekomponente keine anderen Stoffe mehr enthält. Die wässrige Flüssigkeit besteht dann aus Wasser und der Säurekomponente. Die anspruchsgemäße verdünnte Salzsäure besteht jedenfalls aus Wasser und Salzsäure und hat einen sauren pH-Wert, welcher aus einem der zuvor genannten Bereiche ausgewählt sein kann.

Die wässrige Flüssigkeit wird anspruchsgemäß dazu eingesetzt, um das Chitosan in Lösung zu bringen. Dabei wird eine wässrige, chitosanhaltige Zubereitung erhalten, welche typischerweise gleichfalls eine Flüssigkeit ist. Die Farbe und die Viskosität der Zubereitung hängen von der Chitosanart sowie von der Chitosankonzentration ab. Zum Beispiel ist die Zubereitung mit 1 Gew.-% Chitosan klar und dünnflüssig, während die Zubereitung mit 4 Gew.-% Chitosan gelblich und hochviskos ist (bezogen auf ein Chitosan mit geringen molaren Gewicht).

Bei den weiteren Komponenten, die mit dem Präpolymer und der chitosanhaltigen, wässrigen Zubereitung im Herstellungsschritt iii. optional gemischt werden können, kann es sich zum Beispiel um ein Feuchthaltemittel oder ein anorganisches Salz handeln. Damit können die chemischen und physikalischen Eigenschaften des Polymers beeinflusst und für die jeweilige Anwendung angepasst werden. Dennoch ist die Anwesenheit der weiteren Komponenten nicht zwingend erforderlich, weshalb sie vorliegend auch als optional ausgewiesen sind. So kann es vorgesehen sein, dass zum Erhalt der Reaktionsmischung nur das Präpolymer mit der wässrigen, chitosanhaltigen Zubereitung gemischt wird. Die Reaktionsmischung besteht dann aus dem Präpolymer und der wässrigen, chitosanhaltigen Zubereitung.

Als Feuchthaltemittel kann Ethylenglykol, Propylenglykol, PEG300, PEG2000, Glycerol, Saccharose oder Sorbitol verwendet werden. Besonders bevorzugt ist dabei Glycerol. Die Feuchthaltemittel können die Absorptionsfähigkeit des Polymers erhöhen sowie den Feuchtigkeitsverlust des Polymers verringern.

Als anorganisches Salz kann Natriumchlorid, Kaliumchlorid, Magnesiumchlorid, Calciumchlorid oder eine Mischung aus mindestens zwei dieser Salze verwendet werden. Besonders bevorzugt ist dabei Natriumchlorid als alleiniges Salz. Eine bevorzugte Salzmischung enthält Natriumchlorid, Kaliumchlorid und Calciumchlorid. Diese drei Salze werden auch zur Herstellung der Ringerlösung verwendet. Die anorganischen Salze können den Elektrolyt-Gehalt in einem von einer Wunde abgegebenen Wundserum simulieren. Damit stellt das Polymer einer Wunde ein besonders wundheilungsförderndes Klima zur Verfügung.

Die nachfolgenden Bereiche sind erfindungsgemäß für die Herstellung des antibakteriell wirksamen Polymers mit kovalent gebundenen Chitosan vorgesehen:
Der Anteil an dem Präpolymer bezogen auf das Gesamtgewicht der Reaktionsmischung beträgt 5 bis 50 Gew.-%. Insbesondere beträgt der Anteil an dem Präpolymer bezogen auf das Gesamtgewicht der Reaktionsmischung 10 bis 50 Gew.-%.

Der Anteil an dem Chitosan bezogen auf das Gesamtgewicht der Reaktionsmischung beträgt mindestens 0,4 Gew.-%. Insbesondere beträgt der Anteil an dem Chitosan bezogen auf das Gesamtgewicht der Reaktionsmischung 0,4 bis 5 Gew.-%. Der Anteil an dem Chitosan bezogen auf das Gesamtgewicht der Reaktionsmischung kann auch auf einen Bereich von 0,4 bis 1,8 Gew.-% beschränkt sein.

Der Anteil der wässrigen Flüssigkeit bezogen auf das Gesamtgewicht der Reaktionsmischung beträgt 40 bis 90 Gew.-%. Insbesondere beträgt der Anteil der wässrigen Flüssigkeit bezogen auf das Gesamtgewicht der Reaktionsmischung 50 bis 90 Gew.-%.

Der Anteil der einen oder mehreren weiteren Komponenten bezogen auf das Gesamtgewicht der Reaktionsmischung beträgt 0 bis 30 Gew.-%. Der Bereich von 0 bis 30 Gew.-% bezieht sich dabei auf die Summe aller vorhandenen weiteren Komponenten.

Dabei ergeben der Anteil an dem Präpolymer, der Anteil an dem Chitosan, der Anteil der wässrigen Flüssigkeit und der Anteil der weiteren Komponenten zusammen 100 Gew.-%. Wenn keine weiteren Komponenten vorgesehen sind (die optionalen weiteren Komponenten also fehlen), ergeben der Anteil an dem Präpolymer, der Anteil an dem Chitosan

und der Anteil der wässrigen Flüssigkeit zusammen 100 Gew.-%.

Chemisch gesehen handelt es sich bei den hier vorgeschlagenen Polymeren stets um Hydrogele. Ein Hydrogel ist ein disperses System aus mindestens einer festen hydrophilen Phase und einer flüssigen Phase, nämlich Wasser. Dabei bildet die feste Phase ein schwammartiges, dreidimensionales polymeres Netzwerk, welches durch das Wasser ausgefüllt ist. In Abhängigkeit von dem Mengenverhältnis Präpolymer zu wässriger Flüssigkeit in der Reaktionsmischung kann sich das äußere Erscheinungsbild der Polymere jedoch unterscheiden. So kann das Polymer hinsichtlich seines äußeren Erscheinungsbildes als ein Gel, als ein Gelschaum oder als ein Schaumstoff vorliegen.

Wenn das Polymer als ein Gel vorliegt, enthält es keine oder nur wenige Hohlräume beziehungsweise Zellen. Das Polymer kann dann eine zusammenhängende, diskrete Schicht ausbilden. Das Polymer kann als Gel auch transparent sein. Wenn das Polymer als ein Gel vorliegt, weist es also ein für Hydrogele übliches äußeres Erscheinungsbild auf.

Wenn das Polymer als ein Schaumstoff vorliegt, ist es porös und enthält eine Vielzahl offener und/oder geschlossener Hohlräume beziehungsweise Zellen. Das Schaumstoff-Polymer ist typischerweise weiß und undurchsichtig sowie im Vergleich zu einem Gelpolymer mit gleicher Masse voluminöser. Außerdem enthält das Schaumstoff-Polymer gegenüber einem Gelpolymer mit gleicher Masse einen höheren festen polymeren Anteil und weniger Wasser.

Die als "Gelschaum" bezeichnete Variante des Polymers stellt eine Übergangsform zwischen Gel und Schaumstoff dar. Der "Gelschaum" kann auch als ein vorgequollener hydrophiler Schaumstoff aufgefasst werden.

Nachfolgend werden drei alternative Ausführungsformen der Erfindung genannt, in denen die zuvor genannten Bereiche für das Präpolymer und die wässrige Flüssigkeit weiter spezifiziert werden. Infolge der unterschiedlichen Bereiche für das Präpolymer und die wässrige Flüssigkeit unterscheiden sich die Polymere gemäß dieser drei Ausführungsformen in ihrer Konsistenz und liegen entweder als Gel, Gelschaum oder Schaumstoff vor.

Erstens können die Bereiche von dem Präpolymer und der wässrigen Flüssigkeit wie folgt gewählt werden: Der Anteil an dem Präpolymer bezogen auf das Gesamtgewicht der Reaktionsmischung beträgt 10 bis 25 Gew.-%.

Der Anteil der wässrigen Flüssigkeit bezogen auf das Gesamtgewicht der Reaktionsmischung beträgt 74 bis 90 Gew.-%.

Nach dem Umsetzen des Präpolymers in der Reaktionsmischung wird dann ein Polymer in der Form eines Gels erhalten. Das gelartige Polymer hat ein hohes Wasserabgabevermögen und ist somit besonders gut für die Behandlung von trockenen Wunden geeignet.

Zweitens können die Bereiche von dem Präpolymer und der wässrigen Flüssigkeit wie folgt gewählt werden: Der Anteil an dem Präpolymer bezogen auf das Gesamtgewicht der Reaktionsmischung beträgt 26 bis 33 Gew.-%.

Der Anteil der wässrigen Flüssigkeit bezogen auf das Gesamtgewicht der Reaktionsmischung beträgt 66 bis 73 Gew.-%.

Nach dem Umsetzen des Präpolymers in der Reaktionsmischung wird dann ein Polymer im Übergangsbereich Gel zu Schaumstoff erhalten, was vorliegend auch als Gelschaum bezeichnet wird. Der Gelschaum kann sowohl vergleichsweise viel Wasser abgeben als auch vergleichsweise viel Wasser aufnehmen und ist somit für die Behandlung von unterschiedlichsten Wundtypen geeignet.

Drittens können die Bereiche von dem Präpolymer und der wässrigen Flüssigkeit wie folgt gewählt werden: Der Anteil an dem Präpolymer bezogen auf das Gesamtgewicht der Reaktionsmischung beträgt 34 bis 50 Gew.-%.

Der Anteil der wässrigen Flüssigkeit bezogen auf das Gesamtgewicht der Reaktionsmischung beträgt 50 bis 65 Gew.-%.

Nach dem Umsetzen des Präpolymers in der Reaktionsmischung wird dann ein Polymer in der Form eines Schaumstoffs erhalten. Das schaumstoffartige Polymer hat ein hohes Wasseraufnahmevermögen und ist somit besonders gut für die Behandlung von stark exsudierenden Wunden geeignet.

Im Zusammenhang mit der vorliegenden Erfindung ist es ganz besonders vorteilhaft, wenn der Anteil an dem Chitosan bezogen auf das Gesamtgewicht der Reaktionsmischung mindestens 0,8 Gew.-%, insbesondere 0,8 bis 5 Gew.-% oder zumindest 0,8 bis 1,8 Gew.-%, beträgt. Das Polymer entfaltet dann eine hohe antibakterielle Wirksamkeit und kann schnell hergestellt werden.

Falls ein Feuchthaltemittel wie zum Beispiel Glycerol vorgesehen ist, so kann dieses vorteilhafterweise einen Anteil von 10 bis 25 Gew.-%, insbesondere 15 bis 25 Gew.-%, bezogen auf das Gesamtgewicht der Reaktionsmischung haben.

Falls ein anorganisches Salz wie zum Beispiel Natriumchlorid vorgesehen ist, so kann dieses vorteilhafterweise einen Anteil von 0,1 bis 5 Gew.-%, bevorzugt von 0,1 bis 3 Gew.-% und besonders bevorzugt von 0,5 bis 1,5 Gew.-% bezogen auf das Gesamtgewicht der Reaktionsmischung haben.

Bei dem Umsetzen des Präpolymers finden Reaktionen statt, an denen die Isocyanat-Gruppen des Präpolymers beteiligt sind. Das Umsetzen des Präpolymers setzt üblicherweise automatisch ein, sobald die Reaktionsmischung hergestellt worden ist. Das Umsetzen des Präpolymers in der Reaktionsmischung kann als ein Polymerisationsprozess verstanden werden, der zur Ausbildung des Polymers führt.

Typischerweise erfolgt das Umsetzen des Präpolymers in der Reaktionsmischung im Wesentlichen vollständig.

Es liegen dann keine für menschliche und tierische Zellen toxischen Isocyanat-Gruppen in dem Polymer mehr vor. Der Nachweis ob das Präpolymer in der Reaktionsmischung im Wesentlichen vollständig umgesetzt worden ist kann beispielsweise mit Hilfe der IR-Spektroskopie erbracht werden. Eine fehlende Absorptionsbande bei 2260 $cm^{-1}$ für die Isocyanat-Gruppe dient dabei als Nachweis für ein vollständiges Umsetzen des Präpolymers.

An dem Umsetzen des Präpolymers in der Reaktionsmischung können insbesondere die folgenden Reaktionen beteiligt sein:

a) Reaktion einer Isocyanat-Gruppe des Präpolymers mit einer Amin-Gruppe des Chitosans, wobei eine Harnstoff-Bindung gebildet wird.
b) Reaktion einer Isocyanat-Gruppe des Präpolymers mit einer Hydroxyl-Gruppe des Chitosans, wobei eine Urethan-Bindung gebildet wird.
c) Reaktion einer Isocyanat-Gruppe des Präpolymers mit einem Wassermolekül, wobei die Isocyanat-Gruppe unter Freisetzung von Kohlendioxid in eine Amin-Gruppe umgewandelt wird.
d) Reaktion einer Isocyanat-Gruppe des Präpolymers mit einer nach Reaktion c) gebildeten Amin-Gruppe, wobei eine Harnstoff-Bindung gebildet wird.

Reaktion a) läuft schneller ab als Reaktion b) und Reaktion b) läuft wiederum schneller ab als Reaktion c).

Welche der Reaktionen a) bis d) bei dem Umsetzen des Präpolymers in der Reaktionsmischung ablaufen beziehungsweise überwiegen, hängt unter anderem von den zuvor genannten Reaktionsgeschwindigkeiten sowie den Mengenverhältnissen von Präpolymer, Chitosan und Wasser ab.

Insbesondere sind die obigen Reaktionen a) und/oder b) an dem Umsetzen des Präpolymers beteiligt, so dass wie eingangs erwähnt zumindest ein Teil des Chitosans oder vorzugsweise die Gesamtmenge des Chitosans kovalent an das Präpolymer gebunden wird. Falls nur ein Teil des Chitosans kovalent gebunden wird (zum Beispiel wenn wenig Präpolymer und viel Chitosan eingesetzt wird), ist der verbleibende, nicht kovalent gebundene Teil des Chitosans in der Regel gleichfalls fest in die Struktur des Polymers eingeschlossen und kann aus diesem nicht austreten. Hierfür können hydrophile Wechselwirkungen der Chitosanmoleküle mit dem Polymer verantwortlich sein. Nicht kovalent gebundenes Chitosan kann aber auch einfach deshalb in das Polymer eingeschlossen werden, weil die Chitosanmoleküle größer als die "Maschen" des Polymernetzes sind.

Der Nachweis ob das Präpolymer mit dem Chitosan gemäß Reaktion a) oder b) umgesetzt worden ist kann beispielsweise mit Hilfe der IR-Spektroskopie erbracht werden. Hierfür kann die Absorption bei 1654 $cm^{-1}$ (für die Harnstoff-Bindung) beziehungsweise 1714 $cm^{-1}$ (für die Urethan-Bindung) im IR-Spektrum des Polymers ausgewertet werden. Insbesondere kann der kovalente Einbau des Chitosans an einer stärkeren Absorptionsbande bei 1654 $cm^{-1}$ (Harnstoff-Bindung) im Vergleich zu einem Polymer ohne Chitosan erkannt werden.

An dem Umsetzen des Präpolymers in der Reaktionsmischung können also eine oder mehrere Reaktionen ausgewählt aus der Gruppe umfassend Reaktion a), Reaktion b), Reaktion c) und Reaktion d) beteiligt sein, wobei zumindest Reaktion a) und/oder Reaktion b) an dem Umsetzen des Präpolymers beteiligt sind. Die zuvor genannte Gruppe kann hierbei auch noch weitere, an der Umsetzung des Präpolymers beteiligte Reaktionen umfassen.

Es kann vorgesehen sein, dass nur die zuvor genannten Reaktionen a) bis d) an dem Umsetzen des Präpolymers beteiligt sind. An dem Umsetzen des Präpolymers in der Reaktionsmischung wären dann eine oder mehrere Reaktionen ausgewählt aus der Gruppe bestehend aus Reaktion a), Reaktion b), Reaktion c) und Reaktion d) beteiligt, wobei zumindest Reaktion a) und/oder Reaktion b) an dem Umsetzen des Präpolymers beteiligt sind. Das Umsetzen des Präpolymers kann auf die Reaktionen a) bis d) beschränkt sein, wenn die wässrige Flüssigkeit aus Wasser und Säure besteht (zum Beispiel Wasser und Salzsäure, also verdünnte Salzsäure) und die Reaktionsmischung aus dem Präpolymer und der wässrigen, chitosanhaltigen Zubereitung besteht.

Typischerweise ist es weiterhin vorgesehen, dass in der Reaktionsmischung kein Amin-terminiertes Präpolymer, insbesondere kein Amin-terminiertes Präpolymer enthaltend Polyalkylenoxideinheiten, enthalten ist. Derartige Amin-terminierte Präpolymere sind im Handel erhältlich (zum Beispiel als Jeffamin® ED-2003; Huntsman, Everberg, Belgien) und werden anstelle von Chitosan im Stand der Technik häufig mit Isocyanat-terminierten Präpolymeren zur Reaktion gebracht, um Hydrogele zu erhalten. Vorliegend wird aber der Ansatz verfolgt, das Isocyanat-terminierte Präpolymer mit Chitosan und nicht mit einem Amin-terminierten Präpolymer umzusetzen, wobei ein Polymer mit unerwartet vorteilhaften Eigenschaften erhalten werden kann.

Es kann auch vorteilhafterweise vorgesehen sein, dass in der Reaktionsmischung außer dem Chitosan keine weitere Komponente mit einer Amin-Gruppe enthalten ist. Die Reaktionsmischung kann dann auch das zuvor genannte Amin-terminierte Präpolymer nicht enthalten. Wenn die Reaktionsmischung außer dem Chitosan keine weitere Komponente mit einer Amin-Gruppe enthält, kann der kovalente Einbau des Chitosans in das Polymer besonders gut sichergestellt werden.

Das Polymer wurde vorliegend für die Medizin und insbesondere für die Wundbehandlung entwickelt. Entsprechend wird vorliegend mit Anspruch 1 die Verwendung des Polymers in allen seinen Ausführungsformen in der

Wundbehandlung (2. medizinische Indikation) beansprucht. Daran anknüpfend ist im Sinne einer weiteren medizinischen Indikation vor allem die Verwendung des Polymers in allen seinen Ausführungsformen zur Behandlung von infizierten Wunden vorgesehen.

Bei der Verwendung des Polymers in der Wundbehandlung wird dieses typischerweise in einen Wundverband integriert. Entsprechend wird vorliegend mit Anspruch 2 auch ein Wundverband mit dem Polymer in allen seinen Ausführungsformen beansprucht. Ein solcher Wundverband umfasst erfindungsgemäß eine Rückschicht und eine wundkontaktierende Schicht, wobei das Polymer die wundkontaktierende Schicht bildet. Dann kann das Polymer seine vorteilhaften Eigenschaften wie zum Beispiel die Absorption von Wundexsudat und die Abtötung von in dem Wundexsudat enthaltenen infektiösen Mikroorganismen am besten ausüben.

**Beispiele und Figuren**

Mit den nachfolgend beschriebenen Beispielen und Figuren soll die Erfindung exemplarisch näher erläutert werden.

Herstellung der Polymere

Es wurden verschiedene Polymere hergestellt und deren Eigenschaften untersucht. **Tabelle 1** listet die Zusammensetzung und Konsistenz der hergestellten Polymere bestehend aus Präpolymer, Chitosan und verdünnter Salzsäure auf.

Tabelle 1: Zusammensetzung und Konsistenz der hergestellten Polymere

| Beispiel Nummer | Präpolymer [g] | Chitosan [g] | HCl [g] | Präpolymer / Chitosan / HCl Gew.-% | Konsistenz |
|---|---|---|---|---|---|
| 1 | 0,6 | 0,05 | 4,95 | 10,71 / 0,89 / 88,39 | Gel |
| 2 | 0,6 | 0,1 | 4,9 | 10,71 / 1,79 / 87,50 | Gel |
| 3 | 0,8 | 0,05 | 4,95 | 13,79 / 0,86 / 85,34 | Gel |
| 4 | 0,8 | 0,1 | 4,9 | 13,79 / 1,72 / 84,48 | Gel |
| 5 | 1,0 | - | 5 | 16,67 / - / 83,33 | Gel |
| 6 | 1,0 | 0,00625 | 4,99375 | 16,67 / 0,1 / 83,23 | Gel |
| 7 | 1,0 | 0,0125 | 4,9875 | 16,67 / 0,2 / 83,13 | Gel |
| 8 | 1,0 | 0,025 | 4,975 | 16,67 / 0,4 / 82,9 | Gel |
| 9 | 1,0 | 0,05 | 4,95 | 16,67 / 0,83 / 82,5 | Gel |
| 10 | 1,0 | 0,1 | 4,9 | 16,67 / 1,67 / 81,67 | Gel |
| 11 | 1,2 | 0,05 | 4,95 | 19,35 / 0,81 / 79,84 | Gel |
| 12 | 1,2 | 0,1 | 4,9 | 19,35 / 1,61 / 79,03 | Gel |
| 13 | 1,4 | 0,05 | 4,95 | 21,88 / 0,78 / 77,34 | Gel |
| 14 | 1,6 | 0,05 | 4,95 | 24,24 / 0,76 / 75,00 | Gel |
| 15 | 1,6 | 0,1 | 4,9 | 24,24 / 1,52 / 74,24 | Gel |
| 16 | 1,8 | 0,05 | 4,95 | 26,47 / 0,74 / 72,79 | Gelschaum |
| 17 | 2,0 | 0,05 | 4,95 | 28,57 / 0,71 / 70,71 | Gelschaum |
| 18 | 2,0 | 0,1 | 4,9 | 28,57 / 1,43 / 70,00 | Gelschaum |
| 19 | 2,2 | 0,05 | 4,95 | 30,56 / 0,69 / 68,75 | Gelschaum |
| 20 | 2,4 | 0,05 | 4,95 | 32,43 / 0,68 / 66,89 | Gelschaum |
| 21 | 2,4 | 0,1 | 4,9 | 32,43 / 1,35 / 66,22 | Gelschaum |
| 22 | 2,6 | 0,05 | 4,95 | 34,21 / 0,66 / 65,13 | Schaumstoff |
| 23 | 2,8 | 0,05 | 4,95 | 35,90 / 0,64 / 63,46 | Schaumstoff |
| 24 | 2,8 | 0,1 | 4,9 | 35,90 / 1,28 / 62,82 | Schaumstoff |

(fortgesetzt)

| Beispiel Nummer | Präpolymer [g] | Chitosan [g] | HCl [g] | Präpolymer / Chitosan / HCl Gew.-% | Konsistenz |
|---|---|---|---|---|---|
| 25 | 3,0 | 0,05 | 4,95 | 37,50 / 0,63 / 61,88 | Schaumstoff |
| 26 | 3,2 | 0,05 | 4,95 | 39,02 / 0,61 / 60,37 | Schaumstoff |
| 27 | 3,2 | 0,1 | 4,9 | 39,02 / 1,22 / 59,76 | Schaumstoff |
| 28 | 3,4 | 0,05 | 4,95 | 40,48 / 0,60 / 58,93 | Schaumstoff |
| 29 | 3,6 | 0,05 | 4,95 | 41,86 / 0,58 / 57,56 | Schaumstoff |
| 30 | 3,6 | 0,1 | 4,9 | 41,86 / 1,16 / 56,98 | Schaumstoff |
| 31 | 3,8 | 0,05 | 4,95 | 43,18 / 0,57 / 56,25 | Schaumstoff |
| 32 | 4,0 | 0,05 | 4,95 | 44,44 / 0,56 / 55,00 | Schaumstoff |
| 33 | 4,0 | 0,1 | 4,9 | 44,44 / 1,11 / 54,44 | Schaumstoff |
| 34 | 4,2 | 0,05 | 4,95 | 45,65 / 0,54 / 53,80 | Schaumstoff |
| 35 | 4,4 | 0,05 | 4,95 | 46,81 / 0,53 / 52,66 | Schaumstoff |
| 36 | 4,4 | 0,1 | 4,9 | 46,81 / 1,06 / 52,13 | Schaumstoff |
| 37 | 4,6 | 0,05 | 4,95 | 47,92 / 0,52 / 51,56 | Schaumstoff |
| 38 | 4,8 | 0,05 | 4,95 | 48,98 / 0,51 / 50,51 | Schaumstoff |
| 39 | 4,8 | 0,1 | 4,9 | 48,98 / 1,02 / 50,00 | Schaumstoff |
| 40 | 5,0 | 0,05 | 4,95 | 50,00 / 0,50 / 49,50 | Schaumstoff |

Die Herstellung der Polymere soll anhand von Beispiel 9 näher erläutert werden:

Schritt 1 (Herstellung der anspruchsgemäßen wässrigen, chitosanhaltigen Zubereitung): 96 ml demineralisiertes Wasser und 4 ml 1 M Salzsäure wurden gemischt (anspruchsgemäße wässrige Flüssigkeit in Form von verdünnter Salzsäure). Zu dieser Flüssigkeit wurde 1 g Chitosan gegeben und solange gerührt, bis sich das Chitosan vollständig aufgelöst hatte. Entsprechend besaß die Zubereitung eine Chitosankonzentration von 1 Gew.-%.

Schritt 2 (Herstellung der anspruchsgemäßen Reaktionsmischung):

1 g Präpolymer wurden mit 5 g der in Schritt 1 hergestellten Zubereitung gemischt. Die dabei erhaltene Reaktionsmischung bestand demnach wie in Tabelle 1 angegeben aus 1 g Präpolymer, 0,05 g Chitosan und 4,95 g verdünnter Salzsäure. Die Anteile an dem Präpolymer, dem Chitosan und der verdünnten Salzsäure bezogen auf das Gesamtgewicht der Reaktionsmischung betrugen demnach 16,67 Gew.-%, 0,83 Gew.-% beziehungsweise 82,5 Gew.-% (siehe auch Spalte 5 in Tabelle 1 mit der Überschrift "Präpolymer / Chitosan / HCl"). Die Reaktionsmischung wurde in eine Petrischale eingebracht. Es wurden auch größere Mengen der Reaktionsmischung hergestellt, wenn mehrere Petrischalen befüllt werden sollten oder eine Petrischale vollständig mit der Reaktionsmischung zu befüllen war.

Schritt 3 (Erhalt des Polymers):

Die Reaktionsmischung wurde in der Petrischale ruhen gelassen, bis der Polymerisationsprozess und somit die Umsetzung des Präpolymers abgeschlossen war. Der Polymerisationsprozess wurde als abgeschlossen angesehen, wenn die Probe verfestigt war (also den Gelpunkt erreicht hatte) und an ihrer Oberfläche nicht mehr klebrig war. Dies konnte im Labormaßstab mit einem Spatel manuell überprüft werden.

Die anderen in Tabelle 1 aufgeführten Beispiele wurden in ähnlicher Weise wie Beispiel 9 hergestellt. Die Unterschiede bei der Herstellung der anderen Beispiele beschränkten sich im Wesentlichen auf die Chitosankonzentration der in Schritt 1 hergestellten Zubereitung sowie auf die Menge an Präpolymer, die in Schritt 2 mit der chitosanhaltigen Zubereitung gemischt wurde. So enthielt in Beispiel 12 die in Schritt 1 hergestellte Zubereitung 2 Gew.-% Chitosan. 5 g davon wurden dann in Schritt 2 mit 1,2 g Präpolymer gemischt, um die Reaktionsmischung zu erhalten. Wenn die Chitosankonzentration der in Schritt 1 hergestellten Zubereitung größer als 1 Gew.-% sein sollte, musste zum Lösen des Chitosans in der Regel eine größere Menge an 1 M Salzsäure eingesetzt werden (die Salzsäurekonzentration in der wässrigen Flüssigkeit war dann also größer). Im umgedrehten Fall (wenn also die Zubereitung weniger als 1 Gew.-% Chitosan enthalten sollte) konnte gegebenenfalls auch eine geringere Salzsäurekonzentration verwendet werden.

Die folgenden vier Angaben treffen auf alle Beispiele aus Tabelle 1 zu. Auf die Beispiele aus Tabelle 2 und 3 treffen nur die ersten drei Angaben zu.

- Die Flüssigkeit zum Auflösen des Chitosans enthielt nur demineralisiertes Wasser und Salzsäure.
- Das Chitosan hatte ein molares Gewicht von 50 bis 190 kDa und einen Deacetylierungsgrad von 75 bis 85 % (Sigma-Aldrich, Steinheim, Deutschland; Produktnummer 448869).
- Das Präpolymer war Aquapol® PI-13000-31 von Carpenter (Richmond, USA). Wie bereits erwähnt, handelt es sich dabei um ein dreiarmig verzweigtes Isocyanat-terminiertes Präpolymer enthaltend Polyethylenoxid- und Polypropylenoxideinheiten.
- Die Reaktionsmischung enthielt nur das Präpolymer und die wässrige, chitosanhaltige Zubereitung.

Beispiel 5 enthält kein Chitosan und ist daher kein erfindungsgemäßes Polymer. Beispiel 5 diente als lediglich als Kontrollprobe.

Die Polymere aus **Tabelle 2** und **3** enthalten neben Präpolymer, Chitosan und verdünnter Salzsäure noch eine oder mehrere weitere Komponenten wie ein Feuchthaltemittel und/oder ein anorganisches Salz.

Tabelle 2: Zusammensetzung von Polymeren mit Feuchthaltemittel und/oder Salz

| Beispiel Nummer | Feuchthaltemittel | Präpolymer [g] | Chitosan [g] | HCl [g] | Feuchthaltemittel [g] | NaCl [g] |
|---|---|---|---|---|---|---|
| 41 | Ethylenglykol | 1,0 | 0,05 | 4,95 | 1,0 | - |
| 42 | Propylenglykol | 1,0 | 0,05 | 4,95 | 1,0 | - |
| 43 | PEG300 | 1,0 | 0,05 | 4,95 | 1,0 | - |
| 44 | PEG2000 | 1,0 | 0,05 | 4,95 | 1,0 | - |
| 45 | Glycerol | 1,0 | 0,05 | 4,95 | 1,0 | - |
| 46 | Saccharose | 1,0 | 0,05 | 4,95 | 1,0 | - |
| 47 | Sorbitol | 1,0 | 0,05 | 4,95 | 1,0 | - |
| 48 | - | 1,0 | 0,05 | 4,95 | - | 0,1 |
| 49 | Ethylenglykol | 1,0 | 0,05 | 4,95 | 1,0 | 0,1 |
| 50 | Propylenglykol | 1,0 | 0,05 | 4,95 | 1,0 | 0,1 |
| 51 | PEG300 | 1,0 | 0,05 | 4,95 | 1,0 | 0,1 |
| 52 | PEG2000 | 1,0 | 0,05 | 4,95 | 1,0 | 0,1 |
| 53 | Glycerol | 1,0 | 0,05 | 4,95 | 1,0 | 0,1 |
| 54 | Saccharose | 1,0 | 0,05 | 4,95 | 1,0 | 0,1 |
| 55 | Sorbitol | 1,0 | 0,05 | 4,95 | 1,0 | 0,1 |

Tabelle 3: Zusammensetzung der Polymere aus Tabelle 2 in Gew.-% (bezogen auf das Gesamtgewicht der Reaktionsmischung)

| Beispiel Nummer | Präpolymer Gew.-% | Chitosan Gew.-% | HCl Gew.-% | Feuchthaltemittel Gew.-% | NaCl Gew.-% |
|---|---|---|---|---|---|
| 41 - 47 | 14,29 | 0,71 | 70,71 | 14,29 | - |
| 48 | 16,39 | 0,82 | 81,15 | - | 1,64 |
| 49 - 55 | 14,08 | 0,70 | 69,72 | 14,08 | 1,41 |

Die Beispiele 41 bis 55 sind ähnlich zusammengesetzt wie das Beispiel 9. Die Beispiele 41 bis 47 unterscheiden sich von Beispiel 9 dadurch, dass sie zusätzlich ein Feuchthaltemittel enthalten. Das Beispiel 48 enthält im Unterschied zu Beispiel 9 ein anorganisches Salz in Form von Natriumchlorid. Die Beispiele 49 bis 55 enthalten im Unterschied zu Beispiel 9 sowohl ein Feuchthaltemittel als auch ein anorganisches Salz, nämlich Natriumchlorid.

Die Beispiele 41 bis 55 konnten ganz ähnlich wie Beispiel 9 hergestellt werden. Die wässrige, chitosanhaltige Zubereitung wurde wie zuvor in Schritt 1 beschrieben hergestellt. Anschließend wurden 1 g Präpolymer, 5 g der chitosanhaltigen Zubereitung und die weiteren Komponenten (1 g Feuchthaltemittel bei den Beispielen 41-47, 49-55 und 0,1 g Natriumchlorid bei den Beispielen 48-55) miteinander gemischt, in eine Petrischale gegossen und aushärten gelassen. Die verwendeten Feuchthaltemittel sowie das eingesetzte Salz ließen sich aufgrund ihrer hohen Wasserlöslichkeit und insbesondere bei Ansatz größerer Mengen der Reaktionsmischung gut mit dem Präpolymer und der

chitosanhaltigen Zubereitung mischen. Ein größerer Ansatz der Reaktionsmischung konnte bezogen auf Polymer 53 beispielsweise durch Mischen von 10 g Präpolymer, 50 g chitosanhaltige Zubereitung, 10 g Glycerol und 1g Natriumchlorid erhalten werden (= 10-facher Ansatz).

Konsistenz der Polymere

**Figur 1** und **2** zeigen Fotografien ausgewählter Beispiele aus Tabelle 1. Die beiden Figuren sollen veranschaulichen, wie das Polymer als Gel, Gelschaum und Schaumstoff aussehen kann. Die Nummern der Beispiele sind unter den jeweiligen Proben gekennzeichnet.

**Tabelle 4** und **Tabelle 5** führen zur besseren Übersichtlichkeit nochmals die Zusammensetzung und die Konsistenz der Beispiele aus Figur 1 beziehungsweise Figur 2 auf (diese Informationen können aber auch Tabelle 1 entnommen werden).

Tabelle 4: Zusammensetzung und Konsistenz der Polymere aus Figur 1

| Beispiel Nummer | Präpolymer [g] | Chitosan [g] | HCl [g] | Präpolymer / Chitosan / HCl Gew.-% | Konsistenz |
|---|---|---|---|---|---|
| 1 | 0,6 | 0,05 | 4,95 | 10,71 / 0,89 / 88,39 | Gel |
| 3 | 0,8 | 0,05 | 4,95 | 13,79 / 0,86 / 85,34 | Gel |
| 9 | 1,0 | 0,05 | 4,95 | 16,67 / 0,83 / 82,5 | Gel |
| 11 | 1,2 | 0,05 | 4,95 | 19,35 / 0,81 / 79,84 | Gel |
| 13 | 1,4 | 0,05 | 4,95 | 21,88 / 0,78 / 77,34 | Gel |
| 14 | 1,6 | 0,05 | 4,95 | 24,24 / 0,76 / 75,00 | Gel |
| 16 | 1,8 | 0,05 | 4,95 | 26,47 / 0,74 / 72,79 | Gelschaum |
| 17 | 2,0 | 0,05 | 4,95 | 28,57 / 0,71 / 70,71 | Gelschaum |
| 19 | 2,2 | 0,05 | 4,95 | 30,56 / 0,69 / 68,75 | Gelschaum |
| 20 | 2,4 | 0,05 | 4,95 | 32,43 / 0,68 / 66,89 | Gelschaum |
| 22 | 2,6 | 0,05 | 4,95 | 34,21 / 0,66 / 65,13 | Schaumstoff |
| 23 | 2,8 | 0,05 | 4,95 | 35,90 / 0,64 / 63,46 | Schaumstoff |
| 25 | 3,0 | 0,05 | 4,95 | 37,50 / 0,63 / 61,88 | Schaumstoff |
| 26 | 3,2 | 0,05 | 4,95 | 39,02 / 0,61 / 60,37 | Schaumstoff |
| 28 | 3,4 | 0,05 | 4,95 | 40,48 / 0,60 / 58,93 | Schaumstoff |
| 29 | 3,6 | 0,05 | 4,95 | 41,86 / 0,58 / 57,56 | Schaumstoff |
| 31 | 3,8 | 0,05 | 4,95 | 43,18 / 0,57 / 56,25 | Schaumstoff |
| 32 | 4,0 | 0,05 | 4,95 | 44,44 / 0,56 / 55,00 | Schaumstoff |
| 34 | 4,2 | 0,05 | 4,95 | 45,65 / 0,54 / 53,80 | Schaumstoff |
| 35 | 4,4 | 0,05 | 4,95 | 46,81 / 0,53 / 52,66 | Schaumstoff |
| 37 | 4,6 | 0,05 | 4,95 | 47,92 / 0,52 / 51,56 | Schaumstoff |
| 38 | 4,8 | 0,05 | 4,95 | 48,98 / 0,51 / 50,51 | Schaumstoff |
| 40 | 5,0 | 0,05 | 4,95 | 50,00 / 0,50 / 49,50 | Schaumstoff |

Tabelle 5: Zusammensetzung und Konsistenz der Polymere aus Figur 2

| Beispiel Nummer | Präpolymer [g] | Chitosan [g] | HCl [g] | Präpolymer / Chitosan / HCl Gew.-% | Konsistenz |
|---|---|---|---|---|---|
| 2 | 0,6 | 0,1 | 4,9 | 10,71 / 1,79 / 87,50 | Gel |
| 4 | 0,8 | 0,1 | 4,9 | 13,79 / 1,72 / 84,48 | Gel |
| 10 | 1,0 | 0,1 | 4,9 | 16,67 / 1,67 / 81,67 | Gel |
| 12 | 1,2 | 0,1 | 4,9 | 19,35 / 1,61 / 79,03 | Gel |

(fortgesetzt)

| Beispiel Nummer | Präpolymer [g] | Chitosan [g] | HCl [g] | Präpolymer / Chitosan / HCl Gew.-% | Konsistenz |
|---|---|---|---|---|---|
| 15 | 1,6 | 0,1 | 4,9 | 24,24 / 1,52 / 74,24 | Gel |
| 18 | 2,0 | 0,1 | 4,9 | 28,57 / 1,43 / 70,00 | Gelschaum |
| 21 | 2,4 | 0,1 | 4,9 | 32,43 / 1,35 / 66,22 | Gelschaum |
| 24 | 2,8 | 0,1 | 4,9 | 35,90 / 1,28 / 62,82 | Schaumstoff |
| 27 | 3,2 | 0,1 | 4,9 | 39,02 / 1,22 / 59,76 | Schaumstoff |
| 30 | 3,6 | 0,1 | 4,9 | 41,86 / 1,16 / 56,98 | Schaumstoff |
| 33 | 4,0 | 0,1 | 4,9 | 44,44 / 1,11 / 54,44 | Schaumstoff |
| 36 | 4,4 | 0,1 | 4,9 | 46,81 / 1,06 / 52,13 | Schaumstoff |
| 39 | 4,8 | 0,1 | 4,9 | 48,98 / 1,02 / 50,00 | Schaumstoff |

Die Beispiele aus Figur 1 wurden hergestellt, indem eine konstante Menge der chitosanhaltigen Zubereitung mit einer Chitosankonzentration von 1 Gew.-% mit ansteigenden Mengen Präpolymer gemischt wurden. Im Unterschied dazu war die Chitosankonzentration bei den Proben in Figur 2 doppelt so hoch, also 2 Gew.-%.

Der Vergleich der Beispiele aus Figur 1 mit den Beispielen aus Figur 2 hat gezeigt, dass das Mengenverhältnis Präpolymer zu wässriger Flüssigkeit unabhängig von der eingesetzten Chitosanmenge darüber entscheidet, ob ein Polymer in Form eines Geles, eines Gelschaumes oder eines Schaumstoffes erhalten wird. So geht das Polymer von einem gelartigen zu einem schaumstoffartigen Zustand über, wenn die Präpolymerkonzentration in der Reaktionsmischung erhöht wird. Es hat sich weiterhin gezeigt, dass das Chitosan die Festigkeit der erhaltenen Polymere beeinflussen kann. Die Festigkeit der Polymere kann sich erhöhen, wenn der Chitosangehalt in der Reaktionsmischung steigt.

Antibakterielle Wirksamkeit der Polymere

Ausgewählte Beispiele aus Tabelle 1 wurden hinsichtlich ihrer antibakteriellen Wirksamkeit im Weichagartest untersucht. Hierzu wurde eine definierte Konzentration von in infizierten Wunden häufig anzutreffenden Bakterien in Weichagar auf die zu prüfenden Proben aufgegeben und die KBE/ml nach einer Prüfzeit von 24 Stunden ausgewertet (KBE steht für koloniebildende Einheit).

Der Ablauf und die Auswertung des Weichagartests werden im Folgenden kurz erläutert: Der Bakterienstamm wurde zu 9 ml Caso-Boullion gegeben und bei 37°C für 18 bis 24 Stunden inkubiert. Nach der Inkubation betrug die Bakteriendichte der Suspension 1 x $10^8$ KBE/ml.

100 ml Weichagar wurden auf 45 °C temperiert und mit 1 ml der Bakteriensuspension beimpft.

Es wurden Probenstücke der Größe 2,5 cm x 2,5 cm mit einem Stanzeisen ausgestanzt und mit einer sterilen Pinzette in leere Petrischalen überführt.

Je 1 ml des beimpften Weichagars wurde mit einer Pipette auf die Oberfläche der Probenstücke aufgetragen und darauf geachtet, dass der Weichagar nicht herunterfließt. Dieser erstarrt bei Raumtemperatur nach ca. 10 Minuten.

Zusätzlich wurde eine Kontrolle angesetzt. Hierfür wurde 1 ml beimpfter Weichagar in ein 50 ml Falcon-Tube pipettiert und 3 ml 1/4 starke Ringerlösung zugegeben, damit der Weichagar nicht austrocknet.

Die beimpften Probenstücke wurden bei 37°C im Brutschrank inkubiert. Nach Ablauf der Prüfzeit von 24 Stunden wurden 100 ml Dey-Engley-Enthemmer dazu gegeben. Für den 0-Stunden-Wert wurde 1 ml des beimpften Weichagars direkt in den Dey-Engley-Enthemmer pipettiert. Die Ansätze wurden dann eine Minute im Ultraschallbad behandelt und zusätzlich geschüttelt, um die Bakterien in die Lösung freizusetzen.

Anschließend wurden geeignete Verdünnungsreihen mit Dey-Engley-Enthemmer hergestellt und mit einem Spiralplattensystem (Eddy Jet, IUL Instruments) in Caso-Platten ausplattiert. Nach einer Inkubation der Platten bei 37°C für 18 bis 24 Stunden erfolgte die finale Auswertung (ASTM E2180-01). Jede Polymerprobe und jede Kontrolle wurde dreimal im Weichagartest untersucht.

$$\text{Geometrisches Mittel der Probe} = (\log x_1 + \log x_2 + \log x_3)/(3)$$

$$\text{Geometrisches Mittel der Kontrolle} = (\log xk_1 + \log xk_2 + \log xk_3)/(3)$$

$$\text{Log-Stufen Reduktion} = \text{Log-Stufe Kontrolle} - \text{Log-Stufe Probe}$$

**Figur 3** und **Figur 4** zeigen die Ergebnisse der Weichagartests für die Beispiele 8, 9 und 10 beziehungsweise 1 und 17 aus Tabelle 1. Die antibakterielle Aktivität der Proben gegen bis zu drei verschiedene Bakterienstämme, nämlich Staphylococcus aureus (DSM-Nr. 346), Klebsiella pneumoniae (DSM-Nr. 789) und Bacillus subtilis (DSM-Nr. 675), wurde dabei untersucht. Die gestrichelte Linie zeigt die Grenze zwischen einem bakteriostatischen Effekt und einem bakteriziden Effekt an (Werte unterhalb der Linie sind bakteriostatisch, Werte oberhalb der Linie sind bakterizid).

**Tabelle 6** und **Tabelle 7** führen zur besseren Übersichtlichkeit nochmals die Zusammensetzung und die Konsistenz der Beispiele aus Figur 3 beziehungsweise Figur 4 auf (diese Informationen können aber auch Tabelle 1 entnommen werden).

Tabelle 6: Zusammensetzung und Konsistenz der Polymere aus Figur 3

| Beispiel Nummer | Präpolymer [g] | Chitosan [g] | HCl [g] | Präpolymer / Chitosan / HCl Gew.-% | Konsistenz |
|---|---|---|---|---|---|
| 8 | 1,0 | 0,025 | 4,975 | 16,67 / 0,4 / 82,9 | Gel |
| 9 | 1,0 | 0,05 | 4,95 | 16,67 / 0,83 / 82,5 | Gel |
| 10 | 1,0 | 0,1 | 4,9 | 16,67 / 1,67 / 81,67 | Gel |

Tabelle 7: Zusammensetzung und Konsistenz der Polymere aus Figur 4

| Beispiel Nummer | Präpolymer [g] | Chitosan [g] | HCl [g] | Präpolymer / Chitosan / HCl Gew.-% | Konsistenz |
|---|---|---|---|---|---|
| 1 | 0,6 | 0,05 | 4,95 | 10,71 / 0,89 / 88,39 | Gel |
| 17 | 2,0 | 0,05 | 4,95 | 28,57 / 0,71 / 70,71 | Gelschaum |

Wie aus Tabelle 6 entnommen werden kann handelt es sich bei den Beispielen aus Figur 3 um Gele mit einem im Wesentlichen identischen Mengenverhältnis von Präpolymer zu wässriger Flüssigkeit, die sich jedoch in ihrem Chitosangehalt unterscheiden. Anhand dieser Proben sollte die antibakterielle Aktivität unterschiedlicher Chitosankonzentrationen bei ansonsten identischen Polymereigenschaften verglichen werden.

Die Beispiele aus Figur 4 weisen demgegenüber einen im Wesentlichen identischen Chitosangehalt auf, unterscheiden sich aber im Mengenverhältnis von Präpolymer zu wässriger Flüssigkeit und somit in ihrer Konsistenz (siehe Tabelle 7). Anhand dieser Proben sollte untersucht werden, ob die Konsistenz der Polymere deren antibakterielle Aktivität signifikant beeinflusst.

In den Weichagartests konnte eine deutliche antibakterielle Aktivität gegen mindestens einen der Bakterienstämme für alle untersuchten Proben nachgewiesen werden (siehe Figur 3). Eine deutliche antibakterielle Aktivität liegt also bereits ab einem Chitosangehalt von 0,4 Gew.-% vor. Besonders gute Effekte konnten ab einem Chitosangehalt von 0,8 Gew.-% erhalten werden.

Zudem hat sich gezeigt, dass das Mengenverhältnis von Präpolymer zu wässriger Flüssigkeit die antibakterielle Aktivität der Polymere beeinflussen kann. So war die antibakterielle Aktivität der Gelprobe bei dem Bakterienstamm Staphylococcus aureus signifikant besser als die der ansonsten vergleichbaren Gelschaumprobe (siehe Figur 4). Entsprechend werden vorliegend im Hinblick auf die antibakterielle Wirksamkeit die Gelpolymere besonderes bevorzugt.

Reaktionsgeschwindigkeit der Polymere

**Tabelle 8** gibt die Zusammensetzung und die Reaktionsgeschwindigkeit der Beispiele 6 bis 10 aus Tabelle 1 an. Die Reaktionsgeschwindigkeit bezieht sich dabei auf die Zeitspanne, die für das Umsetzen des Präpolymers, also für den Polymerisationsprozess, benötigt wird. Der Polymerisationsprozess läuft mit ansteigender Chitosankonzentration schneller ab. Eine höhere Polymerisationsgeschwindigkeit begünstigt die schnelle, kostengünstige und ökonomische Herstellung der Polymere.

Tabelle 8: Reaktionsgeschwindigkeit (abgekürzt mit RG) der Beispiele 6 bis 10 aus Tabelle 1

| Beispiel Nummer | Präpolymer [g] | Chitosan [g] | HCl [g] | Präpolymer / Chitosan / HCl Gew.-% | RG |
|---|---|---|---|---|---|
| 6 | 1,0 | 0,00625 | 4,99375 | 16,67 / 0,1 / 83,23 | 45 min |
| 7 | 1,0 | 0,0125 | 4,9875 | 16,67 / 0,2 / 83,13 | 20 min |
| 8 | 1,0 | 0,025 | 4,975 | 16,67 / 0,4 / 82,9 | 15 min |
| 9 | 1,0 | 0,05 | 4,95 | 16,67 / 0,83 / 82,5 | 10 min |

(fortgesetzt)

| Beispiel Nummer | Präpolymer [g] | Chitosan [g] | HCl [g] | Präpolymer / Chitosan / HCl Gew.-% | RG |
|---|---|---|---|---|---|
| 10 | 1,0 | 0,1 | 4,9 | 16,67 / 1,67 / 81,67 | 5 min |

Absorptionskapazität der Polymere

Ausgewählte Beispiele aus Tabelle 1 wurden hinsichtlich ihrer Absorptionskapazität untersucht. Zur Messung der Absorptionskapazität wurden Probenstücke mit einer Größe von 2,5 cm x 2,5 cm ausgestanzt und gewogen. Anschließend wurden sie in ein Becherglas mit demineralisiertem Wasser für 24 Stunden gegeben. Daraufhin wurden sie erneut gewogen. Jede Probe wurde dreimal untersucht. Die Berechnung der Absorptionskapazität erfolgt nach folgender Gleichung und wird in der Einheit g/g angegeben:

$$\text{Absorptionskapazität} = (\text{Endgewicht} - \text{Anfangsgewicht}) / \text{Anfangsgewicht}$$

**Figur 5** und **Figur 6** zeigen die Ergebnisse der Absorptionstests für ausgewählte Beispiele aus Tabelle 1.

**Tabelle 9** und **Tabelle 10** führen zur besseren Übersichtlichkeit nochmals die Zusammensetzung und die Konsistenz der Beispiele aus Figur 5 beziehungsweise Figur 6 auf (diese Informationen können aber auch Tabelle 1 entnommen werden).

Tabelle 9: Zusammensetzung und Konsistenz der Polymere aus Figur 5

| Beispiel Nummer | Präpolymer [g] | Chitosan [g] | HCl [g] | Präpolymer / Chitosan / HCl Gew.-% | Konsistenz |
|---|---|---|---|---|---|
| 6 | 1,0 | 0,00625 | 4,99375 | 16,67 / 0,1 / 83,23 | Gel |
| 7 | 1,0 | 0,0125 | 4,9875 | 16,67 / 0,2 / 83,13 | Gel |
| 8 | 1,0 | 0,025 | 4,975 | 16,67 / 0,4 / 82,9 | Gel |
| 9 | 1,0 | 0,05 | 4,95 | 16,67 / 0,83 / 82,5 | Gel |
| 10 | 1,0 | 0,1 | 4,9 | 16,67 / 1,67 / 81,67 | Gel |

Tabelle 10: Zusammensetzung und Konsistenz der Polymere aus Figur 6

| Beispiel Nummer | Präpolymer [g] | Chitosan [g] | HCl [g] | Präpolymer / Chitosan / HCl Gew.-% | Konsistenz |
|---|---|---|---|---|---|
| 1 | 0,6 | 0,05 | 4,95 | 10,71 / 0,89 / 88,39 | Gel |
| 9 | 1,0 | 0,05 | 4,95 | 16,67 / 0,83 / 82,5 | Gel |
| 17 | 2,0 | 0,05 | 4,95 | 28,57 / 0,71 / 70,71 | Gelschaum |
| 25 | 3,0 | 0,05 | 4,95 | 37,50 / 0,63 / 61,88 | Schaumstoff |

Alle untersuchten Proben haben für die Anwendung in der Wundtherapie zufriedenstellende Absorptionskapazitäten gezeigt. Wenn allerdings bei einer definierten Chitosanmenge ein möglichst hohes Absorptionsvermögen erwünscht ist (zum Beispiel wenn stark exsudierende Wunden behandelt werden sollen), können die Gelschaum-Polymere oder insbesondere die Schaumstoff-Polymere den Gelpolymeren vorgezogen werden (siehe Figur 6).

Feuchtigkeitsabgabekapazität der Polymere

Ausgewählte Beispiele aus Tabelle 1 wurden hinsichtlich ihrer Feuchtigkeitsabgabekapazität untersucht. Mit diesem Test konnte bestimmt werden wieviel Feuchtigkeit die Polymere an Filterpapiere abgeben können. Dies kann ein Anhaltspunkt dafür sein, wieviel Feuchtigkeit die Polymere in einem Wundverband an die Wundoberfläche abgeben können.

Der Versuchsablauf sah dabei folgendermaßen aus:
Es wurden zunächst runde Probenstücke mit einem Durchmesser von 3,5 cm aus den hergestellten Polymeren gestanzt.

Jede Probe wurde in eine Petrischale eingebracht, die mit fünf Filterpapieren ausgelegt war und deren Gewicht zuvor ermittelt wurde (Anfangsgewicht). Der Durchmesser von Petrischale und Filterpapier war 5 cm. Die Petrischalen wurden mit Deckeln verschlossen und zusätzlich mit Parafilm abgedichtet, um einen Feuchtigkeitsverlust zu verhindern.

Im Anschluss wurden die Proben bei Raumtemperatur für einen Zeitraum von 24 Stunden in den verschlossenen Petrischalen belassen.

Dann wurden die Petrischalen geöffnet und ohne die Proben erneut gewogen (Endgewicht), um die Feuchtigkeitsabgabekapazität in mg/cm$^2$ berechnen zu können. Jede Probe wurde dreimal untersucht.

$$\text{Feuchtigkeitsabgabekapazität} = ((\text{Endgewicht} - \text{Anfangsgewicht}) \times 1000) / A$$

Anfangsgewicht: Gewicht der Petrischale und der Filterpapiere zu Versuchsbeginn ("trocken") in g
Endgewicht: Gewicht der Petrischale und der Filterpapiere nach dem Versuch ("befeuchtet") in g
A: Fläche der Probe in cm$^2$

**Figur 7** und **Figur 8** zeigen die Ergebnisse der Feuchtigkeitsabgabetests für ausgewählte Beispiele aus Tabelle 1. Es wurden die gleichen Beispiele ausgewählt wie zuvor bei den Absorptionstests. Alle untersuchten Proben haben für die Anwendung in der Wundtherapie zufriedenstellende Feuchtigkeitsabgabekapazitäten gezeigt. Wenn allerdings bei einer definierten Chitosanmenge ein möglichst hohes Feuchtigkeitsabgabevermögen erwünscht ist (zum Beispiel wenn trockene Wunden behandelt werden sollen), können die Gelpolymere den Gelschaum-Polymeren sowie den Schaumstoff-Polymeren vorgezogen werden (siehe Figur 8).

<u>IR-Spektren der Polymere und der Ausgangsstoffe</u>

**Figur 9** zeigt FT-IR-Spektren von ausgewählten Beispielen aus Tabelle 1 sowie von dem eingesetzten Präpolymer (Aquapol®) und Chitosan. In der Figur sind die Spektren folgender Proben zu sehen (von oben nach unten): Chitosan, Präpolymer, Beispiel 5, Beispiel 6, Beispiel 7, Beispiel 8, Beispiel 9, Beispiel 10

Isocyanat-Gruppen, Urethan-Gruppen und Harnstoff-Gruppen führen in den IR-Spektren zu Absorptionsbanden bei 2260 cm$^{-1}$, 1714 cm$^{-1}$ beziehungsweise 1654 cm$^{-1}$. Die aufgenommenen IR-Spektren belegen, dass die hergestellten Polymere keine Isocyanat-Gruppen mehr besitzen und somit eine vollständige Umsetzung der Präpolymere stattfand. Dadurch sind die Polymere gut verträglich für menschliche und tierische Zellen. Das Verhältnis von Urethan-Bande und Harnstoff-Bande hängt von der Zusammensetzung des Polymers ab. Die Polymere mit Chitosan (Beispiele 6 bis 10) haben eine stärkere Harnstoff-Bande (bei 1654 cm$^{-1}$) als das Polymer ohne Chitosan (Beispiel 5). Der Grund hierfür ist der kovalente Einbau des Chitosans in das Polymer unter Ausbildung von zusätzlichen Harnstoff-Bindungen.

<u>Wundverband mit dem Polymer</u>

**Figur 10** zeigt einen Wundverband mit einem erfindungsgemäßen Polymer in schematischer Form. Der Wundverband umfasst eine Rückschicht 1, welche mit einer Klebeschicht 2 vollflächig ausgerüstet ist. Die Rückschicht **1** kann zum Beispiel ein wasserundurchlässiger, wasserdampfdurchlässiger Polyurethanfilm sein, welcher vollflächig mit einem Acrylatkleber als Klebeschicht **2** beschichtet ist. Die Rückschicht **1** dient als tragende und abdeckende Lage in dem Wundverband. Die Klebeschicht **2** ermöglicht die Befestigung des Wundverbands am Körper des Patienten. Weiterhin umfasst der Wundverband eine Wundkontaktschicht **3,** welche von dem erfindungsgemäßen Polymer gebildet wird. Beim Befestigen des Wundverbandes am Körper des Patienten wird die Wundkontaktschicht **3** direkt auf der Wunde platziert. Mit der Wundkontaktschicht **3** kann der Wundverband Wundexsudat absorbieren, Feuchtigkeit an die Wunde abgeben und insbesondere auch eine antibakterielle Aktivität entfalten.

Es ist auch denkbar, dass der Wundverband noch weitere Schichten umfasst. Zum Beispiel kann eine weitere, absorbierende Schicht zwischen der Klebeschicht 2 und der Wundkontaktschicht **3** angeordnet werden, um die Absorptionskapazität des Wundverbandes zu steigern. Die absorbierende Schicht kann dabei beispielsweise einen hydrophilen Polyurethanschaumstoff oder einen Vliesstoff umfassen.

Der Wundverband kann hergestellt werden, indem das Polymer in flüssigem Zustand (also die anspruchsgemäße Reaktionsmischung) auf die Rückschicht **1** beziehungsweise deren Klebeschicht **2** direkt aufgegossen wird. Der fertige Wundverband liegt dann nach dem Aushärten des Polymers vor. Alternativ kann die Wundkontaktschicht **3** auch in einer separaten Gießform hergestellt und dann mit der Rückschicht **1** über deren Klebeschicht **2** verbunden werden.

**Patentansprüche**

1. Polymer zur Anwendung in der Wundbehandlung, wobei das Polymer erhältlich ist durch ein Verfahren umfassend die Schritte

i. Bereitstellen eines Isocyanat-terminierten Präpolymers enthaltend Polyalkylenoxideinheiten,
ii. Lösen eines Chitosans in einer wässrigen Flüssigkeit, um eine wässrige, chitosanhaltige Zubereitung zu erhalten,
iii. Mischen des Präpolymers, der wässrigen, chitosanhaltigen Zubereitung und optional einer oder mehrerer weiterer Komponenten, um eine Reaktionsmischung zu erhalten,
iv. Umsetzen des Präpolymers in der Reaktionsmischung, um das Polymer zu erhalten,

wobei

- der Anteil an dem Präpolymer bezogen auf das Gesamtgewicht der Reaktionsmischung 5 bis 50 Gew.-%, insbesondere 10 bis 50 Gew.-%, beträgt,
- der Anteil an dem Chitosan bezogen auf das Gesamtgewicht der Reaktionsmischung mindestens 0,4 Gew.-%, insbesondere 0,4 bis 5 Gew.-%, beträgt,
- der Anteil der wässrigen Flüssigkeit bezogen auf das Gesamtgewicht der Reaktionsmischung 40 bis 90 Gew.-%, insbesondere 50 bis 90 Gew.-%, beträgt, und
- der Anteil der einen oder mehreren weiteren Komponenten bezogen auf das Gesamtgewicht der Reaktionsmischung 0 bis 30 Gew.-% beträgt.

**2.** Wundverband, umfassend ein Polymer erhältlich durch ein Verfahren umfassend die Schritte

i. Bereitstellen eines Isocyanat-terminierten Präpolymers enthaltend Polyalkylenoxideinheiten,
ii. Lösen eines Chitosans in einer wässrigen Flüssigkeit, um eine wässrige, chitosanhaltige Zubereitung zu erhalten,
iii. Mischen des Präpolymers, der wässrigen, chitosanhaltigen Zubereitung und optional einer oder mehrerer weiterer Komponenten, um eine Reaktionsmischung zu erhalten,
iv. Umsetzen des Präpolymers in der Reaktionsmischung, um das Polymer zu erhalten,

wobei

- der Anteil an dem Präpolymer bezogen auf das Gesamtgewicht der Reaktionsmischung 5 bis 50 Gew.-%, insbesondere 10 bis 50 Gew.-%, beträgt,
- der Anteil an dem Chitosan bezogen auf das Gesamtgewicht der Reaktionsmischung mindestens 0,4 Gew.-%, insbesondere 0,4 bis 5 Gew.-%, beträgt,
- der Anteil der wässrigen Flüssigkeit bezogen auf das Gesamtgewicht der Reaktionsmischung 40 bis 90 Gew.-%, insbesondere 50 bis 90 Gew.-%, beträgt, und
- der Anteil der einen oder mehreren weiteren Komponenten bezogen auf das Gesamtgewicht der Reaktionsmischung 0 bis 30 Gew.-% beträgt, und

wobei der Wundverband eine Rückschicht (1) und eine wundkontaktierende Schicht (3) umfasst, wobei das Polymer die wundkontaktierende Schicht (3) bildet.

**3.** Polymer oder Wundverband nach Anspruch 1 oder 2, wobei das Präpolymer mindestens dreiarmig verzweigt ist und die Polyalkylenoxideinheiten durch Polyethylenoxid- und/oder Polypropylenoxideinheiten gebildet werden, wobei das Gewichtsverhältnis von Ethylenoxid- zu Propylenoxideinheiten vorzugsweise 3 : 1 bis 7 : 1 beträgt.

**4.** Polymer oder Wundverband nach einem der vorangehenden Ansprüche, wobei das Chitosan

- ein molares Gewicht von 50 kDa bis 375 kDa, bevorzugt von 50 kDa bis 310 kDa und besonders bevorzugt von 50 kDa bis 190 kDa aufweist und/oder
- einen Deacetylierungsgrad von mindestens 50 %, bevorzugt von mindestens 60 %, mehr bevorzugt von mindestens 70 %, besonders bevorzugt von mindestens 75 % und insbesondere von 75 % bis 85 % aufweist.

**5.** Polymer oder Wundverband nach einem der vorangehenden Ansprüche, wobei die wässrige Flüssigkeit einen sauren pH-Wert aufweist und insbesondere verdünnte Salzsäure ist.

**6.** Polymer oder Wundverband nach einem der vorangehenden Ansprüche, wobei ein Feuchthaltemittel als weitere Komponente vorhanden ist, wobei das Feuchthaltemittel vorzugsweise Ethylenglykol, Propylenglykol, PEG300, PEG2000, Glycerol, Saccharose oder Sorbitol ist.

7. Polymer oder Wundverband nach einem der vorangehenden Ansprüche, wobei ein anorganisches Salz als weitere Komponente vorhanden ist, wobei das Salz vorzugsweise Natriumchlorid, Kaliumchlorid, Magnesiumchlorid, Calciumchlorid oder eine Mischung aus mindestens zwei dieser Salze ist.

8. Polymer oder Wundverband nach einem der vorangehenden Ansprüche, wobei das Polymer als ein Gel, ein Gelschaum oder ein Schaumstoff vorliegt.

9. Polymer oder Wundverband nach einem der vorangehenden Ansprüche 1 bis 8, wobei

- der Anteil an dem Präpolymer bezogen auf das Gesamtgewicht der Reaktionsmischung 10 bis 25 Gew.-% beträgt, und
- der Anteil der wässrigen Flüssigkeit bezogen auf das Gesamtgewicht der Reaktionsmischung 74 bis 90 Gew.-% beträgt.

10. Polymer oder Wundverband nach einem der vorangehenden Ansprüche 1 bis 8, wobei

- der Anteil an dem Präpolymer bezogen auf das Gesamtgewicht der Reaktionsmischung 26 bis 33 Gew.-% beträgt, und
- der Anteil der wässrigen Flüssigkeit bezogen auf das Gesamtgewicht der Reaktionsmischung 66 bis 73 Gew.-% beträgt.

11. Polymer oder Wundverband nach einem der vorangehenden Ansprüche 1 bis 8, wobei

- der Anteil an dem Präpolymer bezogen auf das Gesamtgewicht der Reaktionsmischung 34 bis 50 Gew.-% beträgt, und
- der Anteil der wässrigen Flüssigkeit bezogen auf das Gesamtgewicht der Reaktionsmischung 50 bis 65 Gew.-% beträgt.

12. Polymer oder Wundverband nach einem der vorangehenden Ansprüche, wobei der Anteil an dem Chitosan bezogen auf das Gesamtgewicht der Reaktionsmischung mindestens 0,8 Gew.-%, insbesondere 0,8 bis 5 Gew.-%, beträgt.

**Claims**

1. Polymer for use in wound treatment, wherein the polymer is obtainable by a process comprising the steps of

i. providing an isocyanate-terminated prepolymer containing polyalkylene oxide units,
ii. dissolving a chitosan in an aqueous liquid to obtain an aqueous, chitosan-containing preparation,
iii. mixing the prepolymer, the aqueous, chitosan-containing preparation and optionally one or more further components to obtain a reaction mixture,
iv. reacting the prepolymer in the reaction mixture to obtain the polymer,

wherein

- the proportion of the prepolymer based on the total weight of the reaction mixture is 5% to 50% by weight, in particular 10% to 50% by weight,
- the proportion of the chitosan based on the total weight of the reaction mixture is at least 0.4% by weight, in particular 0.4% to 5% by weight,
- the proportion of the aqueous liquid based on the total weight of the reaction mixture is 40% to 90% by weight, in particular 50% to 90% by weight, and
- the proportion of the one or more further components based on the total weight of the reaction mixture is 0% to 30% by weight.

2. Wound dressing, comprising a polymer obtainable by a process comprising the steps of

i. providing an isocyanate-terminated prepolymer containing polyalkylene oxide units,
ii. dissolving a chitosan in an aqueous liquid to obtain an aqueous, chitosan-containing preparation,
iii. mixing the prepolymer, the aqueous, chitosan-containing preparation and optionally one or more further

components to obtain a reaction mixture,
iv. reacting the prepolymer in the reaction mixture to obtain the polymer,

wherein

- the proportion of the prepolymer based on the total weight of the reaction mixture is 5% to 50% by weight, in particular 10% to 50% by weight,
- the proportion of the chitosan based on the total weight of the reaction mixture is at least 0.4% by weight, in particular 0.4% to 5% by weight,
- the proportion of the aqueous liquid based on the total weight of the reaction mixture is 40% to 90% by weight, in particular 50% to 90% by weight, and
- the proportion of the one or more further components based on the total weight of the reaction mixture is 0% to 30% by weight, and

wherein the wound dressing comprises a backing layer (1) and a wound-contacting layer (3), wherein the polymer forms the wound-contacting layer (3).

**3.** Polymer or wound dressing according to Claim 1 or 2, wherein the prepolymer has at least three-armed branching and the polyalkylene oxide units are formed by polyethylene oxide units and/or polypropylene oxide units, wherein the weight ratio of ethylene oxide units to propylene oxide units is preferably 3:1 to 7:1.

**4.** Polymer or wound dressing according to any of the preceding claims, wherein the chitosan

- has a molar weight of 50 kDa to 375 kDa, preferably from 50 kDa to 310 kDa and particularly preferably from 50 kDa to 190 kDa and/or
- has a degree of deacetylation of at least 50%, preferably of at least 60%, more preferably of at least 70%, particularly preferably of at least 75% and in particular of 75% to 85%.

**5.** Polymer or wound dressing according to any of the preceding claims, wherein the aqueous liquid has an acidic pH and in particular is dilute hydrochloric acid.

**6.** Polymer or wound dressing according to any of the preceding claims, wherein a humectant is present as further component, wherein the humectant is preferably ethylene glycol, propylene glycol, PEG300, PEG2000, glycerol, sucrose or sorbitol.

**7.** Polymer or wound dressing according to any of the preceding claims, wherein an inorganic salt is present as further component, wherein the salt is preferably sodium chloride, potassium chloride, magnesium chloride, calcium chloride or a mixture of at least two of these salts.

**8.** Polymer or wound dressing according to any of the preceding claims, wherein the polymer is in the form of a gel, a gel foam or a foam.

**9.** Polymer or wound dressing according to any of the preceding Claims 1 to 8, wherein

- the proportion of the prepolymer based on the total weight of the reaction mixture is 10% to 25% by weight, and
- the proportion of the aqueous liquid based on the total weight of the reaction mixture is 74% to 90% by weight.

**10.** Polymer or wound dressing according to any of the preceding Claims 1 to 8, wherein

- the proportion of the prepolymer based on the total weight of the reaction mixture is 26% to 33% by weight, and
- the proportion of the aqueous liquid based on the total weight of the reaction mixture is 66% to 73% by weight.

**11.** Polymer or wound dressing according to any of the preceding Claims 1 to 8, wherein

- the proportion of the prepolymer based on the total weight of the reaction mixture is 34% to 50% by weight, and
- the proportion of the aqueous liquid based on the total weight of the reaction mixture is 50% to 65% by weight.

**12.** Polymer or wound dressing according to any of the preceding claims, wherein the proportion of the chitosan based on

the total weight of the reaction mixture is at least 0.8% by weight, in particular 0.8% to 5% by weight.

**Revendications**

1. Polymère destiné à être utilisé dans le traitement de plaies, le polymère pouvant être obtenu par un procédé comprenant les étapes suivantes

    i. mise à disposition d'un prépolymère terminé par isocyanate contenant des motifs poly(oxyde d'alkylène),
    ii. dissolution d'un chitosane dans un liquide aqueux afin d'obtenir une préparation aqueuse, contenant du chitosane,
    iii. mélange du prépolymère, de la préparation aqueuse contenant du chitosane et éventuellement d'un ou de plusieurs composants afin d'obtenir un mélange réactionnel,
    iv. transformation du prépolymère dans le mélange réactionnel, afin d'obtenir le polymère,

    dans lequel

    - la proportion de prépolymère par rapport au poids total du mélange réactionnel représente 5 à 50% en poids, en particulier 10 à 50% en poids,
    - la proportion de chitosane par rapport au poids total du mélange réactionnel représente au moins 0,4% en poids, en particulier 0,4 à 5% en poids,
    - la proportion de liquide aqueux par rapport au poids total du mélange réactionnel représente 40 à 90% en poids, en particulier 50 à 90% en poids et
    - la proportion dudit un ou desdits plusieurs autres composants par rapport au poids total du mélange réactionnel représente 0 à 30% en poids.

2. Pansement, comprenant un polymère pouvant être obtenu par un procédé comprenant les étapes suivantes

    i. mise à disposition d'un prépolymère terminé par isocyanate contenant des motifs poly(oxyde d'alkylène),
    ii. dissolution d'un chitosane dans un liquide aqueux afin d'obtenir une préparation aqueuse, contenant du chitosane,
    iii. mélange du prépolymère, de la préparation aqueuse contenant du chitosane et éventuellement d'un ou de plusieurs composants afin d'obtenir un mélange réactionnel,
    iv. transformation du prépolymère dans le mélange réactionnel, afin d'obtenir le polymère,

    dans lequel

    - la proportion de prépolymère par rapport au poids total du mélange réactionnel représente 5 à 50% en poids, en particulier 10 à 50% en poids,
    - la proportion de chitosane par rapport au poids total du mélange réactionnel représente au moins 0,4% en poids, en particulier 0,4 à 5% en poids,
    - la proportion de liquide aqueux par rapport au poids total du mélange réactionnel représente 40 à 90% en poids, en particulier 50 à 90% en poids et
    - la proportion dudit un ou desdits plusieurs autres composants par rapport au poids total du mélange réactionnel représente 0 à 30% en poids, et

    le pansement comprenant une couche arrière (1) et une couche (3) en contact avec la plaie, le polymère formant la couche (3) en contact avec la plaie.

3. Polymère ou pansement selon la revendication 1 ou 2, dans lequel le prépolymère est ramifié en au moins trois bras et les motifs de poly(oxyde d'alkylène) sont formés par des motifs de poly(oxyde d'éthylène) et/ou des motifs de poly(oxyde de propylène), le rapport en poids des motifs d'oxyde d'éthylène aux motifs d'oxyde de propylène représentant de préférence 3: 1 à 7: 1.

4. Polymère ou pansement selon l'une quelconque des revendications précédentes, dans lequel le chitosane

    - présente un poids molaire de 50 kDa à 375 kDa, de préférence de 50 kDa à 310 kDa et de manière particulièrement préférée de 50 kDa à 190 kDa et/ou

- présente un degré de désacétylation d'au moins 50 %, de préférence d'au moins 60 %, plus préférablement d'au moins 70 %, de manière particulièrement préférée d'au moins 75 % et en particulier de 75 % à 85 %.

**5.** Polymère ou pansement selon l'une quelconque des revendications précédentes, dans lequel le liquide aqueux présente un pH acide et est en particulier de l'acide chlorhydrique dilué.

**6.** Polymère ou pansement selon l'une quelconque des revendications précédentes, dans lequel un agent de rétention d'humidité est présent comme autre composant, l'agent de rétention d'humidité étant de préférence l'éthylèneglycol, le propylèneglycol, le PEG300, le PEG2000, le glycérol, le saccharose ou le sorbitol.

**7.** Polymère ou pansement selon l'une des revendications précédentes, dans lequel un sel inorganique est présent comme autre sel, le sel étant de préférence le chlorure de sodium, le chlorure de potassium, le chlorure de magnésium, le chlorure de calcium ou un mélange d'au moins deux de ces sels.

**8.** Polymère ou pansement selon l'une quelconque des revendications précédentes, le polymère se présentant sous forme de gel, de mousse de gel ou de mousse.

**9.** Polymère ou pansement selon l'une quelconque des revendications 1 à 8 précédentes, dans lequel

- la proportion de prépolymère par rapport au poids total du mélange réactionnel représente 10 à 25% en poids et
- la proportion de liquide aqueux par rapport au poids total du mélange réactionnel représente 74 à 90% en poids.

**10.** Polymère ou pansement selon l'une quelconque des revendications 1 à 8 précédentes, dans lequel

- la proportion de prépolymère par rapport au poids total du mélange réactionnel représente 26 à 33% en poids et
- la proportion de liquide aqueux par rapport au poids total du mélange réactionnel représente 66 à 73% en poids.

**11.** Polymère ou pansement selon l'une quelconque des revendications 1 à 8 précédentes, dans lequel

- la proportion de prépolymère par rapport au poids total du mélange réactionnel représente 34 à 50% en poids et
- la proportion de liquide aqueux par rapport au poids total du mélange réactionnel représente 50 à 65% en poids.

**12.** Polymère ou pansement selon l'une quelconque des revendications précédentes, dans lequel la proportion de chitosane par rapport au poids total du mélange réactionnel repréente au moins 0,8 % en poids, en particulier 0,8 à 5 % en poids.

Figur 1

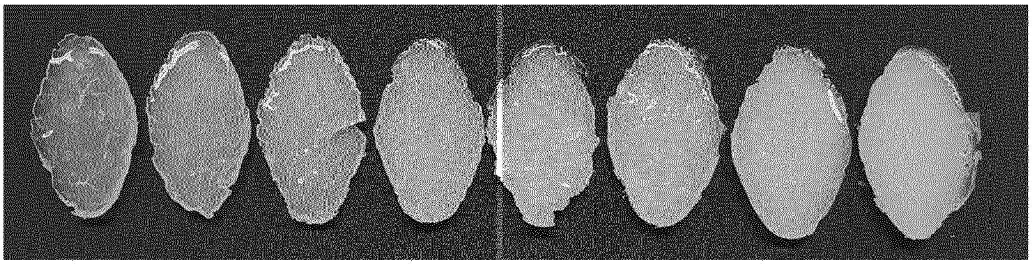

1 3 9 11 13 14 16 17

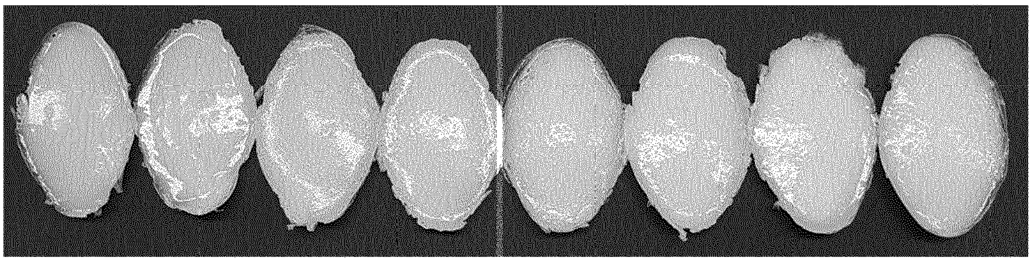

19 20 22 23 25 26 28 29

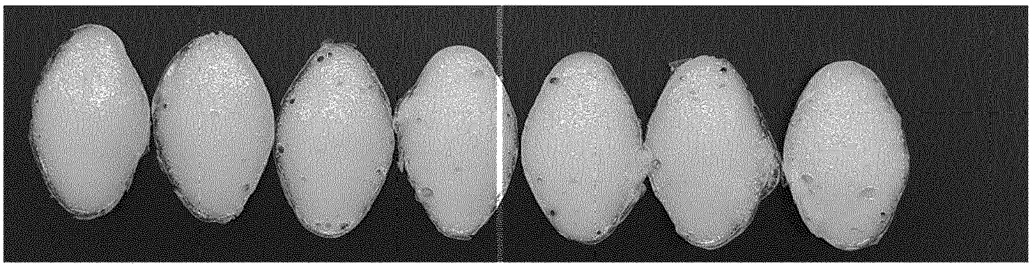

31 32 34 35 37 38 40

Figur 2

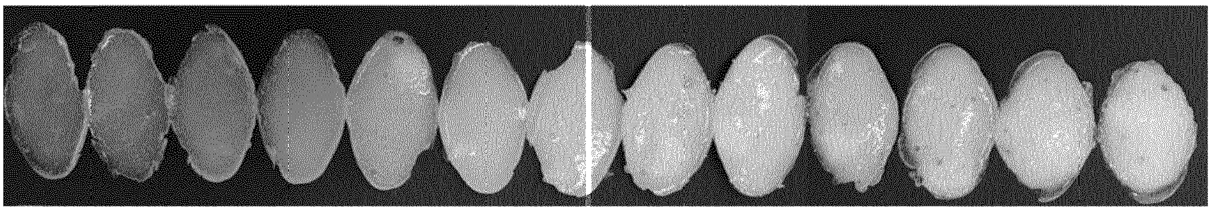

2 4 10 12 15 18 21 24 27 30 33 36 39

Figur 3

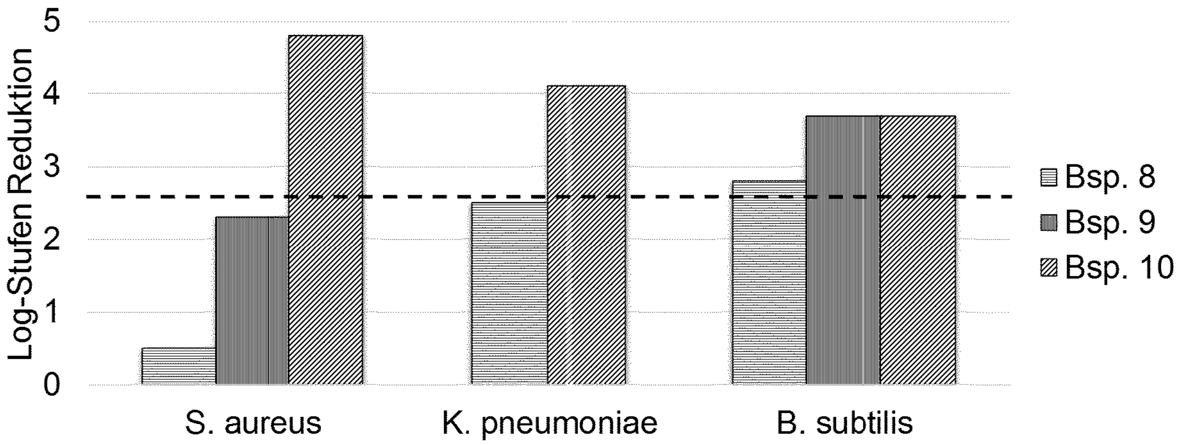


Figur 4

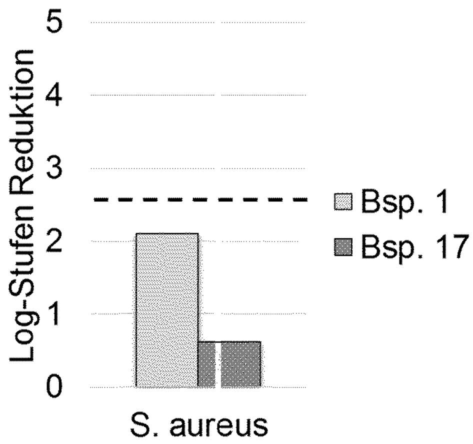

Figur 5

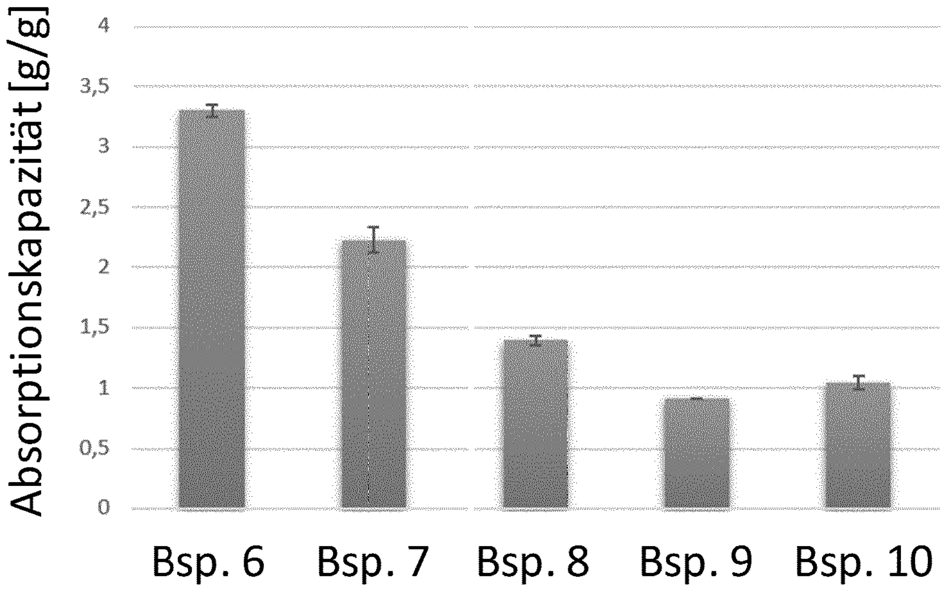


Figur 6

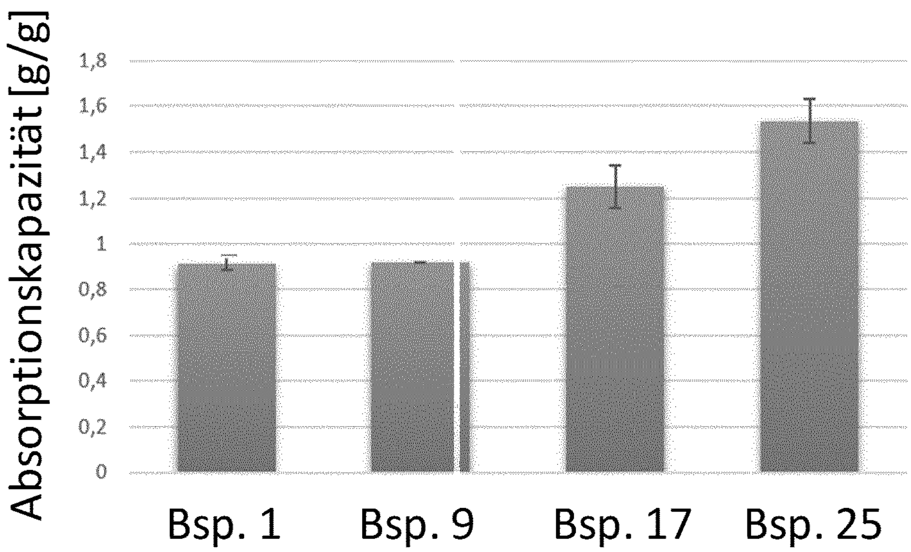

Figur 7

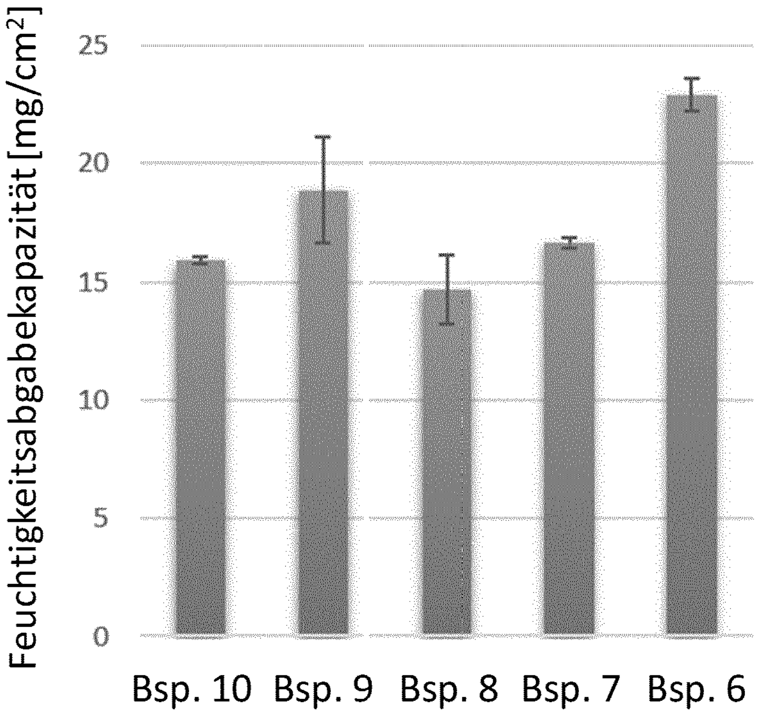


Figur 8

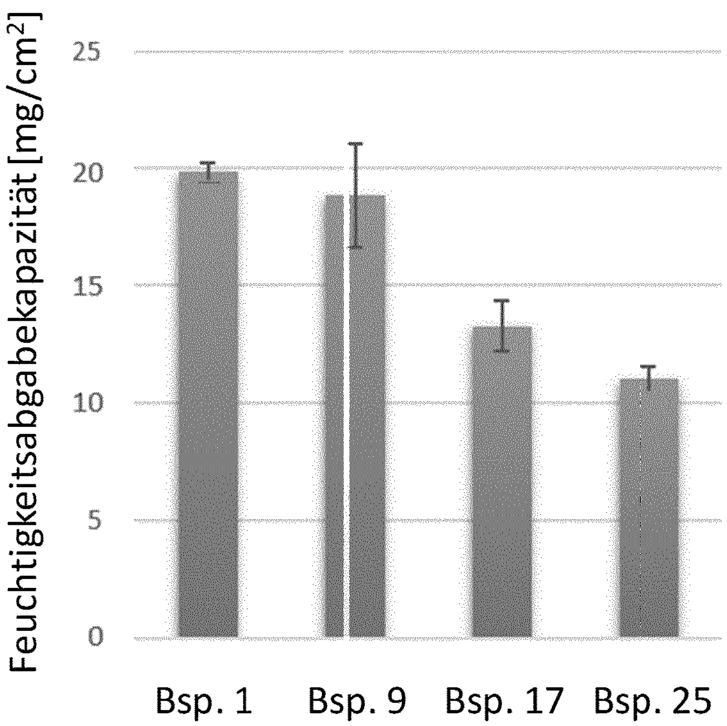

Figur 9

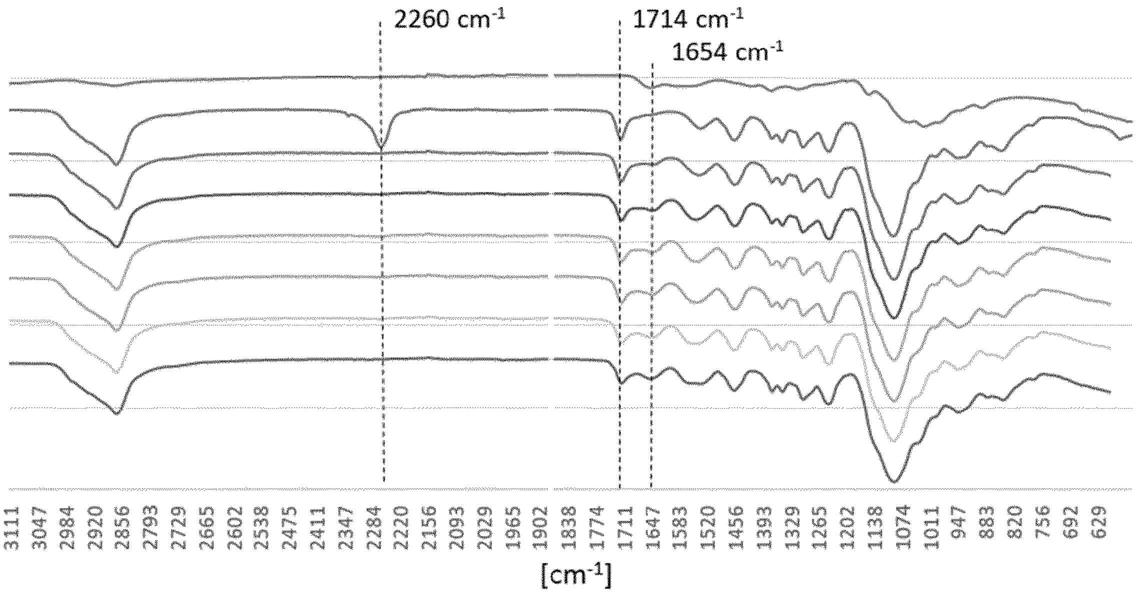


Figur 10

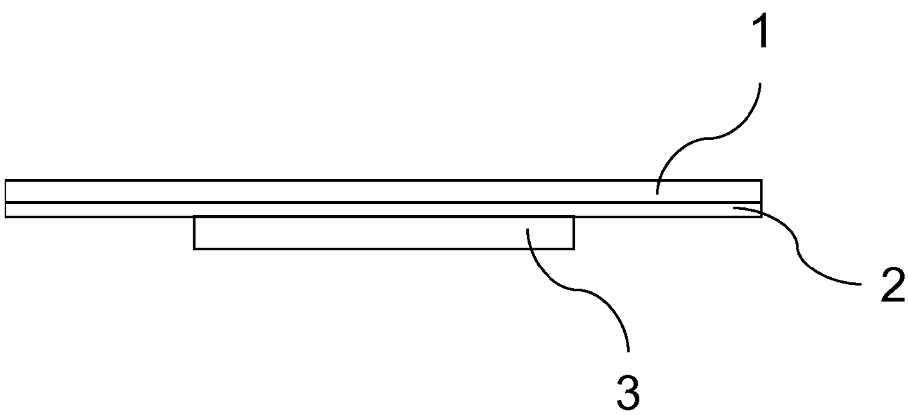

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Nicht-Patentliteratur


- **VELAZQUEZ-MORALES et al.** Polymer electrolytes derived from chitosan/polyether networks. *Electrochimica Acta*, 1998, vol. 43
- **GANDINI et al.** Recent contributions to the preparation of polymers derived from renewable resources. *Journal of Polymers and the Environment*, 2002, vol. 10
- **LEVI-POLYACHENKO et al.** Chitosan wound dressing with hexagonal silver nanoparticles for hyperthermia and enhanced delivery of small molecules. *Colloids and Surfaces B: Biointerfaces*, 2016, vol. 142